# EUROPÄISCHE PATENTSCHRIFT

(11) **EP 2 986 985 B1**
(45) Veröffentlichungstag und Bekanntmachung des Hinweises auf die Patenterteilung: **03.01.2018**
(21) Anmeldenummer: 14734721.5
(22) Anmeldetag: 17.04.2014
(51) Int. Cl.: G01N 33/50, G01N 33/52, G01N 33/53

(54) **AUTOMATISIERBARES VERFAHREN ZUR IDENTIFIZIERUNG, QUANTIFIZIERUNG UND DISKRIMINIERUNG VON SPEZIFISCHEN SIGNALEN GEGENÜBER UNSPEZIFISCHEN SIGNALEN IN NACHWEISVERFAHREN MITTELS DETEKTOR**
AUTOMATABLE METHOD FOR THE IDENTIFICATION, QUANTIFICATION AND DISCRIMINATION OF SPECIFIC SIGNALS IN RELATION TO NON-SPECIFIC SIGNALS IN DETECTION METHODS BY MEANS OF A DETECTOR
PROCÉDÉ AUTOMATISABLE POUR IDENTIFIER, QUANTIFIER ET DISTINGUER DES SIGNAUX SPÉCIFIQUES PAR RAPPORT À DES SIGNAUX NON SPÉCIFIQUES DANS DES PROCÉDÉS DE DÉTECTION AU MOYEN D'UN DÉTECTEUR

(30) Priorität: 19.04.2013 DE 102013006714
(43) Veröffentlichungstag der Anmeldung: 24.02.2016
(73) Patentinhaber: Zyagnum AG, 60528 Frankfurt am Main (DE)
(72) Erfinder: COY, Johannes F., 63512 Hainburg (DE)
(74) Vertreter: CH Kilger Anwaltspartnerschaft mbB
(86) Internationale Anmeldenummer: PCT/DE2014/000214
(87) Internationale Veröffentlichungsnummer: WO 2014/169896

(56) Entgegenhaltungen:
- US-A1- 2012 015 001
- ALEXANDER HAHN ET AL: "Immunolabelling of Atrazine Residues in Soil", ZEITSCHRIFT FÜR PFLANZENERNÄHRUNG UND BODENKUNDE, Bd. 155, Nr. 3, 1. Januar 1992 (1992-01-01), Seiten 203-208, XP055143163, ISSN: 0044-3263, DOI: 10.1002/jpln.19921550308
- STJERNSCHANTZ J ET AL: "Localization of prostanoid receptors and cyclo-oxygenase enzymes in Guinea pig and human cochlea", HEARING RESEARCH, ELSEVIER SCIENCE PUBLISHERS, AMSTERDAM, NL, Bd. 197, Nr. 1-2, 1. November 2004 (2004-11-01), Seiten 65-73, XP004622514, ISSN: 0378-5955, DOI: 10.1016/J.HEARES.2004.04.018
- XIUZHU WANG ET AL: "Electrostatic Orientation of Enzymes on Surfaces for Ligand Screening Probed by Force Spectroscopy", LANGMUIR, Bd. 22, Nr. 3, 1. Januar 2006 (2006-01-01) , Seiten 887-892, XP055143158, ISSN: 0743-7463, DOI: 10.1021/la0525731
- SPENCE M M ET AL: "DEVELOPMENT OF A FUNCTIONALIZED XENON BIOSENSOR", JOURNAL OF THE AMERICAN CHEMICAL SOCIETY, ACS PUBLICATIONS, US, Bd. 126, 1. Januar 2004 (2004-01-01), Seiten 15287-15294, XP001222077, ISSN: 0002-7863, DOI: 10.1021/JA0483037
- SABINE PL PRG A[PARAGRAPH]TTNER ET AL: "Analysis of CYP1A1 induction in single cells of urothelial cell populations by flow cytometry", ANALYTICAL AND BIOANALYTICAL CHEMISTRY, SPRINGER, BERLIN, DE, vol. 392, no. 6, 18 September 2008 (2008-09-18), pages 1149-1158, XP019652913, ISSN: 1618-2650, DOI: 10.1007/S00216-008-2363-7

## Beschreibung

Die Erfindung betrifft ein Verfahren zur Identifizierung, Quantifizierung und Diskriminierung von spezifischen Signalen gegenüber unspezifischen Signalen ("Hintergrundsignalen") in einem Nachweisverfahren, das unter Einsatz wenigstens eines Detektors zum Auffinden und Identifizieren (zur Detektion) bestimmter Zielstrukturen an (zuvor gewonnenem) Probenmaterial durchgeführt wird, wobei der Detektor Signale zu seinem Nachweis (d.h. zum Nachweis des Detektors) abgibt (emittiert) oder generiert (z.B. über enzymatische Reaktionen) und diese Signale eine Quantifizierung des Detektors ermöglichen, und wobei die spezifischen Signale diejenigen Signale sind, die der Detektor infolge Auffindens und Identifizierens der Zielstruktur abgibt (emittiert) oder generiert.
Der Nachweis von Krankheiten oder anderen unerwünschten Konditionen kann durch den Nachweis von Strukturen vorgenommen werden, die spezifisch für die Krankheit oder die unerwünschte Kondition sind, z.B. als Ursache oder als Epiphänomen.
Verfahren zum Nachweis (Auffinden und Identifizieren) solcher Strukturen basieren oftmals auf der Interaktion der zu detektierenden Struktur mit einem Detektor.
Ein Detektor kann z.B. ein Antikörper sein, der ein Epitop eines Proteins erkennt, oder auch eine anorganisch hergestellte Detektorsubstanz sein, die nach dem Schlüssel-Schloss-Prinzip spezifisch mit der zu detektierenden Struktur interagiert und diese damit nachweisbar macht.
Ein entscheidender Faktor für die Qualität solcher Nachweisverfahren ist die Spezifität mit der die Struktur durch den Detektor erkannt wird. Je höher die Spezifität desto besser kann mit dem Testverfahren zwischen positivem und negativem Testergebnis diskriminiert werden.
Jede Interaktion zwischen Detektor und zu detektierender Struktur ist durch ein bestimmtes Maß an Spezifität charakterisiert. Je höher die Spezifität dieser Interaktion, desto geringer ist die Rate an unspezifischer Detektion. Aber auch wenn die Spezifität sehr hoch ist, kommt es immer noch zu unspezifischen Interaktionen zwischen Detektor und Struktur, die zu falsch positiven Testergebnissen führen. Diese unspezifische Interaktion kann von dem Teil des Detektors herrühren, der direkt mit der Struktur interagiert, oder aber von Bestandteilen des Detektors verursacht werden, die nicht direkt mit der Struktur interagieren.

Bei Testverfahren, bei denen z.B. ein monoklonaler Antikörper zur Detektion eines bestimmten Epitopes eines bestimmtem Peroteins an oder in einer Zelle (z.B. Tumorzelle) eingesetzt wird, setzt sich das Gesamtergebnis zusammen aus dem spezifischen Signal, das durch die spezifische Bindung der variablen Region des Antikörpers mit dem zu detektierenden Epitop des Proteins resultiert, und unspezifischen Signalen. Die unspezifischen Signale resultieren z.B. aus Interaktionen der variablen Region des Antikörpers mit ähnlichen Epitopen andere Proteine oder aus Interaktionen der konstanten Region des Antikörpers mit Strukturen der Zelle, z.B. mit Rezeptoren.
Werden Fluoreszenzfarbstoffe zur Markierung und Sichtbarmachung des Detektors verwendet, besteht das Problem, dass viele Zellen eine Eigenfluoreszenz zeigen, die zudem in Abhängigkeit von der Wellenlänge des anregenden Lichts verschieden sein kann. Auch diese Eigenfluoreszenz der Zellen verfälscht das detektierte Gesamtsignal.
Eine weitere Ursache von unspezifischen Signalen besteht in der unspezifischen Bindung (Klebrigkeit) von Antikörpern an andere Zellstrukturen oder - im Fall von intrazellulären Markierungen - in einem unvollständigen Auswaschen von nicht gebundenen Antikörpern aus dem Zytoplasma.

Um das Ausmaß der unspezifischen Bindungen und sonstigen unspezifischen Signale zu bestimmen, müssen immer Kontrollen durchgeführt werden.

Eine Form der Bestimmung von unspezifischen Signalen besteht in der Durchführung eines parallelen Testverfahrens ohne Zugabe desjenigen Antikörpers, der das Zielantigen (die Zielstruktur) erkennt, die sogenannte FMO(Fluorescence-minus-one)-Kontrolle.
Mit der FMO-Kontrolle werden solche unspezifischen Signale erfasst, die durch die Eigenfluoreszenz der Zelle und/oder durch unerwünschte Fluoreszenz (unerwünschte Nebenemissionen) der eingesetzten Farbstoffe im Zielkanal (d.h. im Detektionskanal für die Lichtwellenlängen, die von dem am Zielepitop bzw. an der Zielstruktur gebundenen Detektionsantikörper emittiert wird) erzeugt werden.

Solche FMO-Kontrollen werden oftmals für nichtklebrige Zelltypen wie z.B. T-Zellen verwendet. Hierdurch ist es möglich, im Bereich des emittierten Lichtes desjenigen Fluoreszenzfarbstoffes, der für den Nachweis des Zielantigens verwendet wird, denjenigen Anteil der Lichtintensität zu bestimmen, der auch ohne Anwesenheit des Detektionsantikörpers vorhanden ist/wäre.

Eine andere Form der Bestimmung von unspezifischen Signalen im Fall von Nachweisverfahren mittels Detektionsantikörpern besteht in der Durchführung einer sogenannten Isotypkontrolle. Hiefür wird in einem parallelen Kontrollansatz das betreffende Nachweisverfahren mit einem Isotyp des Detektionsantikörpers durchgeführt, d.h. mit einem Antikörper des gleichen Subtyps wie der spezifische Detektionsantikörper, allerdings gerichtet gegen ein Antigen bzw. Epitop, das in dem Organismus, der oder dessen Zellen in dem Nachweisverfahren untersucht werden, nicht vorkommt.
Ein Signal, das durch Zugabe des Isotypkontroll-Antikörpers erzeugt wird, steht für ein unspezifisches Signal, das allein infolge des "Anklebens" des Antikörpers an Zellstrukturen generiert wird.
Die Isotypkontrolle wird regelmäßig insbesondere dann durchgeführt, wenn es sich bei den zu untersuchenden Zellen (die hinsichtlich des Zielantigens analysiert werden) um sogenannte klebrige Zellen wie z.B. Monozyten, Dendritischen Zellen, oder B-Zellen handelt.

Sowohl die FMO-Kontrolle als auch die Isotypkontrolle zeigen jedoch jeweils nur einen Teil des gesamten unspezifischen Signals auf.
Eine simultane Durchführung von FMO-Kontrolle und Isotypkontrolle in demselben Probenansatz ist nicht möglich, und damit auch keine genaue Bestimmung aller unspezifischen Bindungen.

Beim Nachweisverfahren für intrazelluläre Strukturen unter Einsatz von Detektionsantikörpern oder anderen Detektoren, die intrazellulär binden und damit markieren, kommt es zu weiteren unspezifischen Bindungen, die noch deutlich schwieriger zu bestimmen sind, als beim Nachweis von Strukturen auf der Oberfläche von Zellen.

Dieses Problem tritt bereits bei dem Nachweis einer einzigen intrazellulären Struktur auf und wird noch größer, wenn z.B. zwei intrazelluläre Strukturen zeitgleich detektiert werden sollen.
Bei diesen als "intrazelluläre Färbung" bezeichneten Verfahren werden mittels eines Permeabilisierungspuffers Löcher in der Zellmembran generiert, durch die die markierten Detektoren, beispielsweise Detektionsantikörper, in die Zelle gelangen können. Die Permeabilisierungsparameter, d.h. die Dauer der Präsenz der Löcher in der Zellmembran und die Größe dieser Löcher in der Zellmembran, müssen sehr genau und eng begrenzt gewählt werden, um eine tödliche Schädigung der Zelle - zum Beispiel durch Ausströmen von Zytoplasmaflüssigkeit mit wichtigen Zellbestandteilen - zu vermeiden, um gleichzeitig aber genügend Antikörper in die Zelle einschleusen zu können und nach ausreichender Bindungszeit ungebundene Antikörper aus der Zelle auch wieder auswaschen zu können.

Die Analyse solcher intrazellulären Färbungen wird im Fall von fluoreszenzmarkierten Detektoren, insbesondere fluoreszenzmarkierten Detektionsantikörpern, üblicherweise mittels Durchflusszytometrie oder Fluoreszenzmikroskopie durchgeführt. Dabei wird die Fluoreszenz und somit die Präsenz des Antikörpers bzw. Detektors bestimmt, unabhängig davon, ob der Antikörper/Detektor an seine Zielstruktur (das Epitop) gebunden hat oder nicht. Mit anderen Worten: Die bloße Präsenz des Detektors/Antikörpers - unabhängig von seiner Bindung an die Zielstruktur - wird über seine emittierte Fluoreszenz als positives Signal gewertet. Es wird nicht diskriminiert zwischen Detektoren/Antikörpern, die die Zielstruktur gebunden haben, und solchen, die frei im Zytoplasma vorliegen oder unspezifisch gebunden haben. Ungebundene oder unspezifisch gebundene Detektoren/Antikörpern tragen erheblich zum Ausmaß der unspezifischen Signale bei, weil bei intrazellulären Färbungen (im Unterschied zu Färbungen von Strukturen auf Zelloberflächen oder Gewebeschnitten) nicht effizient gewaschen werden kann, um selbst schwache unspezifische Bindungen wieder aufzulösen und um ungebundenen Detektor/Antikörper aus der Zelle weitgehend auszuwaschen.
Aufgrund all dessen stellt das detektierte Fluoreszenzsignal eines intrazellulären Detektors/Antikörpers immer eine Mischung aus spezifischen Signalen und unspezifischen Signalen dar. Wie hoch der Anteil an unspezifischen Signalen dabei ist, lässt sich bisher nicht quantifizieren, weil nicht zwischen spezifischen und unspezifischen Signalen diskriminiert werden kann.

Verfahren zur Identifizierung, Quantifizierung und Diskriminierung von spezifischen Signalen gegenüber unspezifischen Signalen, wurden bereits offenbart (Alexander Hahn et. al, 1992; Sthernschantz et. al, 2004; Phipps Richard P et. al US 2012/015001 A1; Xiuzhu Wang et. al, 2006; and Spence M M et. al, 2004). Darin werden Detektoren (z.B. Antikörper) zum Auffinden und Identifizieren bestimmter Zielstrukturen an Probenmaterial genutzt. Der Detektor generiert und gibt Signale ab, welche nachgewissen und quantifiziert werden können, wobei die spezifischen Signale diejenigen Signale sind, die der Detektor infolge Auffindens und Identifizierens der Zielstruktur abgibt oder generiert. Diese Verfahren bestehen aus dem eigentlichen Testansatz und wenigstens einem Kontrollansatz. Der Kontrollansatz enthält dieselben Komponenten in den gleichen Mengen wie der parallel gehandhabte eigentliche Testansatz und unterscheidet sich vom eigentlichen Testansatz dadurch, dass die Bindedomäne(n) des Detektors/der Detektoren blockiert ist/sind z.B. mit spezifischen Peptiden. Die Gesamtheit der gemessenen Signale im Kontrollansatz wird von der Gesamtheit der gemessenen Signale im eigentlichen Testansatz subtrahiert, wobei die Differenz das spezifische Signal des Detektors/der Detektoren für die nachzuweisende(n) Zielstruktur(en) darstellt.

Diese Verfahren können für verschiedene Messtechniken verwendet werden, allerdings nicht in Durchflusszytometrie. Eine Studie von 2008 (Sabine Plöttner et. al, 2008), offenbart ein durchflusszytometrisches Verfahren, in dem Hintergrund-Signale der unspezifischen Bindungen, die spezifischen Signale stören. In der Studie, wurde eine Korrektur auf Basis anderer Marker als Kontrollansatz ausgeführt, allerdings war es dadurch nicht möglich die unspezifische Fluoreszenz völlig zu eliminieren. Ein Verfahren, welches das Verhältnis von Signal zu Hintergrund-Signal mittels Durchflusszytometrie verbessert ist somit bislang unbekannt.

Aufgabe der vorliegenden Erfindung ist die Verbesserung von Nachweisverfahren, die auf der Interaktion zwischen Detektor und Zielstruktur beruhen, dahingehend, dass die spezifischen Interaktionen zwischen Detektor und Zielstruktur genauer bestimmbar sind, weil die spezifischen positiven Signale (Testergebnisse) einfacher und zuverlässiger von unspezifischen Signalen (Testergebnissen) diskriminiert werden können.
Eine Lösung dieser Aufgabe besteht in der Bereitstellung eines Verfahrens der eingangs genannten Art, das dadurch gekennzeichnet ist, dass (i) wenigstens ein eigentlicher Testansatz und wenigstens ein Kontrollansatz bereitgestellt werden, die parallel gehandhabt werden, dass (ii) der Kontrollansatz dieselben Komponenten in den gleichen Mengen wie der parallel gehandhabte eigentliche Testansatz enthält und sich von diesem eigentlichen Testansatz (vorzugsweise allein) dadurch unterscheidet, dass die Bindedomäne(n) des Detektors bzw. der Detektoren blockiert ist bzw. sind, vorzugsweise durch eine Substanz (einem Stoff; einem Mittel), die der Zielstruktur gleicht, und dass (iii) die Gesamtheit (bzw. Summe) der gemessenen Signale im Kontrollansatz von der Gesamtheit (bzw. Summe) der gemessenen Signalen im eigentlichen Testansatz subtrahiert wird, wobei die Differenz das spezifische Signal des Detektors /der Detektoren für die nachzuweisende(n) Zielstruktur(en) darstellt, wobei der Nachweistest ein durchflusszytometrisches Untersuchungsverfahren ist und mittels Durchflusszytometrie durchgeführt wird. (Mit anderen Worten: durch eine Vergleichsmessung zwischen blockiertem und nichtblockiertem Detektor wird das spezifische Signal des Detektors errechnet.).
Mit anderen Worten: Die Lösung dieser Aufgabe besteht darin, (i) ein Parallelexperiment durchzuführen, bei dem der Detektor vor seinem Einsatz mit der zu erkennenden Struktur blockiert wird, und (ii) das Testergebnis, das mit dem blockierten Detektor generiert wird (d.h. den "Kontrollwert"), mit demjenigen Testergebnis zu vergleichen, das mit dem unblockierten Detektor generiert wird (d.h. mit dem "Testwert"). (iii) Die Differenz zwischen beiden, d.h. Testwert minus Kontrollwert, gibt den Wert an, der für die spezifische Bindung des Detektors an die Zielstruktur bzw. an das zu erkennende Substrat steht.

Als Detektoren kommen insbesondere Antikörper, vorzugsweise monoklonale Antikörper in Betracht.

Wenn als Detektor ein monoklonaler Antikörper verwendet wird, kann dieser "Detektionsantikörper" für seinen Einsatz in dem Kontrollansatz an seiner Bindedomäne mit einem Peptid blockiert werden, das seinem Ziel-Epitop entspricht, so dass er das Ziel-Epitop in dem Probenmaterial nicht mehr binden kann. Mit anderen Worten: Im Fall von Detektionsantikörpern kann deren jeweilige Bindedomaine mit einem "Blocker-Peptid" so abgesättigt werden, dass die Ziel-Epitope (in der Probe des Kontrollansatzes) nicht mehr gebunden und folglich nicht mehr detektiert werden können.
Als ein solches "Blocker-Peptid" eignet sich insbesondere ein Peptid, das die Aminosäuresequenz der Zielstruktur, d.h. hier des Ziel-Epitops, aufweist (d.h. diese Aminosäuresequenz vollständig oder nahezu vollständig umfasst oder aus ihr besteht). Als "Blocker-Peptid" kommen außerdem in Betracht (i) Peptide oder Proteinfragmente, die das Ziel-Epitop (die Aminosäuresequenz der Zielstruktur) aufweisen (d.h. aus ihr bestehen oder sie umfassen), oder (ii) ganze Proteine, die das Ziel-Epitop (die Aminosäuresequenz der Zielstruktur) tragen (umfassen), oder (iii) ein oder mehrere Peptid-Kompetitoren, der/die mit dem Peptid, das die Aminosäuresequenz des Ziel-Epitops aufweisen (d.h. aus ihr bestehen oder sie umfassen), funktions- und wirkungsgleich ist/sind, und der/die an die Bindedomäne(n) des/der "Detektionsantikörper(s)") bindet/binden, oder auch (iv) eine Kombination aus einem oder mehreren Peptid-Kompetitoren und/oder Proteinfragment(en) und/oder Protein(en), wobei jeder Peptid-Kompetitor mit dem Peptid, das die Aminosäuresequenz der Zielstruktur aufweist, funktions- und wirkungsgleich ist, und jeder Proteinfragment- oder Proteinkompetitor die Zielstruktur umfasst.

Die folgenden Begriffe haben im Kontext der vorliegenden Beschreibung folgende Bedeutung:
Detektion = Auffinden/Erkennen und Anzeigen
Bindedomaine = Region an/auf dem Detektor (z.B. dem Antikörper), in der die Zielstruktur (z.B. das bestimmte Epitop des nachzuweisenden Proteins) gebunden wird.
Detektor = Stoff/Substanz/Mittel, der/die/das eine Zielstruktur spezifisch erkennt und an seiner Bindedomaine mit dieser Zielstruktur interagiert (vorzugsweise bindet).
Detektionsantikörper = Antikörper, vorzugsweis monoklonaler Antikörper, der als Detektor zum Auffinden/Erkennen und Binden an die Zielstruktur (hier insbesondere ein bestimmtes Epitop eines bestimmten Proteins) verwendet wird.
Blocker-Peptid = Peptid, das die Bindedomaine des Detektionsantikörpers blockiert, so dass das Ziel-Epitop nicht mehr binden kann.
Spezifisches Signal = das Signal, das der Detektor bzw. der an diesen Detektor gekoppelte Marker abgibt (emittiert), wenn (sobald) er seine Zielstruktur aufgefunden und daran angekoppelt/gebunden hat.
Testansatz (Synonym: "eigentlicher Testansatz" = (Proben-)Ansatz mit "such- und nachweisfähigem" Detektor, dessen Bindedomäne für die Zielstruktur nicht (zuvor) blockiert wurde.
Kontrollansatz = (Proben-)Ansatz mit den gleichen Komponenten in den gleichen Mengen wie der (eigentliche) Testansatz, wobei jedoch der Detektor bei Zugabe zu dem Ansatz an seiner Bindedomäne für die Zielstruktur blockiert ist, d.h. zuvor an dieser Bindedomaine gezielt blockiert wurde.

Mit dem erfindungsgemäßen Verfahren ist es möglich, auf höchst einfache Weise gezielt zwischen spezifischen und unspezifischen Interaktionen (Bindungen) des Detektors zu unterscheiden und zu bestimmen, welche der erfassten Detektorsignale spezifisch sind und welche unspezifisch sind. Damit geht eine signifikante Steigerung der betreffenden Nachweisverfahren einher, verbunden mit einer wesentlich höheren Sicherheit der Diagnose.

Im Fall eines Detektionsantikörpers werden mit dem erfindungsgemäßen Verfahren insbesondere auch solche unspezifischen Bindungen erfasst, die von dem konstanten Anteil des Detektionsantikörpers oder von einem Zweitantikörper verursacht werden, und/oder die von Farbstoffen oder Enzymen ausgehen, die an die Antikörper gekoppelten sind.

Das Ausmaß der unspezifischen Bindungen kann auch inter-individuell (d.h. innerhalb eines Organismus) unterschiedlich sein, und auch unspezifische Signale aufgrund solcher inter-individuellen Unterschiede können mit dem erfindungsgemäß Verfahren erkannt und berücksichtigt werden. Zum Beispiel kann die erhöhte Konzentration eines Proteins XY und eine damit verbundene unspezifische Bindung des Antikörpers an dieses Protein zu einem Anstieg des unspezifischen Signals führen. Unterliegt die Konzentration dieses Proteins XY inter-individuellen Schwankungen, so kann das bei Anwendung der herkömmlichen Testverfahren mit Kontrollansätzen, bei denen die Probe von einem gesunden Organismus/Individuum B stammen, zu falschen Ergebnissen führen, weil ein 10 erhöhtes Signal im Testansatz mit Proben von Individuum A im Vergleich zum Kontrollansatz mit Proben vom Individuum B als spezifisches Signal gewertet wird und nicht auf ein erhöhtes unspezifisches Signal zurückgeführt wird, das durch die unspezifische Bindung an das in höherer Konzentration vorliegende Protein XY zustande gekommen ist. Beispielsweise können immunologische Reaktionen wie Heuschnupfen oder eine Grippeerkrankungen dazu fuhren, dass der Anteil bestimmter Immunzellen innerhalb des Blutes ansteigt, so dass der/die Detektionsantikörper vermehrt mit solchen klebrigen Zellen (z.B. Makrophagen) in Kontakt kommen und unspezifische Bindungen mit diesen eingehen, wodurch sich der Anteil an unspezifischen Signalen individuell erhöht.
Mit dem erfindungsgemäßen Verfahren werden in den Parallelexperimenten mit blockierten Detektionsantikörpern (den "Kontrollansätzen") auch diese unspezifischen Signale erfasst. Durch Subtraktion des Gesamtmesswerts der unspezifischen Signale dieser Kontrollansätze von dem Gesamtmesswerts der Signale der eigentlichen Testansätze wird der Messwert für die spezifischen Signale errechnet und das Ausmaß der spezifischen Bindungen individuell bestimmt. Insbesondere bei schwachen spezifischen Signalen oder selten vorkommenden Zelltypen (z.B. Tumorzellen) kann so die Spezifität des betreffenden Nachweisverfahrens deutlich gesteigert werden.

Bevorzugte Ausführungsvarianten des offenbarten Verfahrens bestehen darin, dass der Nachweistest ein durchflusszytometrisches Untersuchungsverfahren ist und mittels Durchflusszytometrie durchgeführt wird, oder dass der Nachweistest ein fluoreszenzmikroskopisches Untersuchungsverfahren ist und mittels Fluoreszenzmikroskopie durchgeführt wird, oder dass der Nachweistest eine Kombination aus durchflusszytometrischem und fluoreszenzmikrospopischem Untersuchungsverfahren ist und mittels Durchflusszytometrie und Fluoreszenzmikroskopie durchgeführt wird.
Mit der Einführung dieser Kontrollmessung in Nachweisverfahren, insbesondere in die bekannten immunhistochemischen und immunzytologischen Nachweisverfahren mit Fluoreszenzmikroskopie und/oder Durchflusszytrometrie als Analysemethode, lassen sich diese verlässlicher und sicherer durchführen. Die Auswertung der Testergebnisse ist wesentlich vereinfacht, weil die beiden Messungen von (eigentlichem) Testansatz und Kontrollansatz direkt miteinander verglichen bzw. voneinander abgezogen werden können. Zudem lässt sich die Sensitivität des betreffenden Tests enorm steigern, weil durch die Messung der Hintergrundsignale (der unspezifischen Bindungen) in dem Kontrollansatz und Subtraktion dieser Messwerte von den Messwerten mit dem eigentliche Testansatz das spezifische Signal errechnet werden kann und somit praktisch das Verhältnis von Signal zu Hintergrund verbessert wird.
So lassen sich z.B. auch effizient und einfach solche Zellen im Blut bestimmen, die nur in geringer Zellzahl vorkommen, z.B. zirkulierende Tumorzellen oder besonders seltene Immunzellen. Monozyten oder Makrophagen lassen sich ebenfalls deutlich besser detektieren und analysieren, weil der Anteil der unspezifischen Signale determiniert und vom Gesamtsignal abgezogen werden kann.
Im Fall von Proben, die von (mutmaßlich) erkrankten Spenderorganismen (Patienten) stammen, und insbesondere dann, wenn schon im Vorfeld vermutet werden kann, dass das Ausmaß der unspezifischen Signale recht hoch ist, und/oder die unspezifischen Signal individuell schwanken und/oder durch Veränderungen im Spenderorganismus, insbesondere in dessen Immunsystem, beeinflusst sind/werden (wie z.B. infolge von Erkältungen, Heuschnupfen, Cortisoneinnahme), bietet die Anwendung des erfindungsgemäßen Verfahrens große Vorteile: Weil diese unspezifischen Signale separat erfasst und gemessen werden, und anhand dieser Messwerte der Messwert für das/jedes spezifische Signal mit hoher Genauigkeit errechnet werden kann, sind die Ergebnisse der Nachweisverfahren genauer, sicherer und zuverlässiger, und ebenso die darauf basierenden Diagnosen.

Im Fall von immunzytologischen Nachweisverfahren mit intrazellulären Färbungen ergibt sich mit dem offenbarten Nachweisverfahren folgende Situation:
Nach der zeitweisen Generierung von Löcher in der Zellmembran, dem Einschleusen des Peptid-geblockten fluoreszenzgefärbten Antikörpers in die Zelle, dem anschließenden Waschen und Schließen der Poren, verbleibt ein gewisser Anteil des fluoreszenzmarkierten Detektionsantikörpers in der Zelle (teilweise ungebunden aber nicht ausgewaschen und teilweise unspezifisch gebunden). Seine emittierten Signale zeigen das Ausmaß der unspezifischen Bindung an.
Parallel hierzu wird der nicht-blockierte fluoreszenzgefärbte Antikörper in ansonsten identischer Weise in einem ansonsten identischen Experiment eingesetzt. Durch die spezifische Bindung des Antikörpers an das Ziel-Epitop wird eine feste Bindung zwischen Antikörper und Ziel-Epitop erzeugt, die nicht durch Waschen oder Diffusion gelöst wird. Doch auch hier kommt es zu unspezifischen Bindungen des Antikörpers an zelluläre Strukturen, so dass unspezifische Signale generiert werden.
Aufgrund der spezifischen Bindungen des Antikörpers und dem damit verbundenen weniger starken Auswaschen aus der Zelle ist das Gesamtsignal allerdings höher als das in der Parallelmessung mit blockiertem Antikörper. Von dem Gesamtsignal der Messung des Testansatzes mit unblockiertem Antikörper ("Testmessung") wird das Signal der Parallelmessung in dem Kontrollansatz mit dem blockierten Antikörper ("Kontrollmessung") abgezogen, und nur die übrig bleibenden Messwerte sind als spezifische Messsignale zu bewerten.
Im Fall von immunhistochemischen und immunzytologischen Nachweisverfahren ergibt sich mit dem offenbarten Verfahren folgende Situation: Bei den immunhistochemischen und immunzytologischen Nachweisverfahren wird parallel zu den eigentlichen Testansätzen, bei denen die Detektionsantikörpern mit "freier", d.h. unbesetzter bzw. unblockierter Bindungsdomäne eingesetzt werden, als Kontrolle bzw. Kontroll-Test eine Vergleichsmessung an (wenigstens) einem Kontroll-Ansatz durchgeführt, der sich (vorzugsweise nur) dadurch von dem betreffenden parallelen "eigentlichen" Testansatz unterscheidet, dass der/jeder Detektionsantikörper an seiner Bindungsdomäne mit einem Peptid oder einem in der Wirkung vergleichbaren (d.h. funktions- und wirkungsgleichen oder wirkungsähnlichen) Kompetitor gekoppelt bzw. abgesättigt ist, so dass eine spezifische Bindung des Antikörpers an "sein" Epitop in/an der (im Untersuchungsfokus stehenden) Zielstruktur verhindert ist. Die Messung der Antikörper-Signale in/an diesem Kontroll-Ansatz und ihr Vergleich mit den gemessenen Antikörpersignalen im eigentlichen Test-Ansatz ermöglicht die Bestimmung der unspezifischen und der spezifischen Signale des konkreten Testverfahrens: Das Gesamtsignal des eigentlichen Testansatzes minus (abzüglich/nach Abzug) der unspezifischen Signale im Kontrollansatz liefert den Wert für das spezifische Signal der im Untersuchungsfokus stehenden Zielstruktur.

Zwar ist es bekannt, dass es bei immunhistologischen Nachweisverfahren unter Einsatz von Antikörpern als Detektoren möglich ist, unspezifische Signale von spezifischen Signalen dadurch zu diskriminieren, dass der Antikörper "geblockt" wird, z.B. mit einem Peptid, doch die immunhistologische Untersuchung wird dann nicht mit parallelen Test- und Kontrollansätzen (Testansatz mit unblockiertem Antikörper und Kontrollansatz mit blockiertem Antikörper) durchgeführt. Es wird damit kein Messwert für die unspezifischen Signale ermittelt, und es erfolgt keine Differenzbestimmung zur Errechnung des Messwerts für die spezifischen Signale (spezifischen Signalwerts). Stattdessen werden nur Messungen an Proben-Ansätzen mit unblockiertem Antikörper durchgeführt, und die erhaltenen Ergebnisse in Form der gemessenen Signale des Antikörpers stellen folglich immer die Mischung aus Signalen von spezifisch gebundenem und von unspezifisch gebundenem Antikörper dar.

Das erfindungsgemäße Verfahren kann auch problemlos mit teil-automatischen (teilweise automatischen) oder vollautomatischen Auswertungsmethoden kombiniert werden. Im Stand der Technik werden nicht nur in der Durchflusszytometrie, sondern inzwischen auch häufig in der Immunhistochemie die Ergebnisse von Nachweisverfahren mit immunhistochemischen Färbungen digital erfasst und ausgewertet. Bei Anwendung des erfindungsgemäßen Verfahrens mit Parallelbestimmung des unspezifischen Signals ist ein direkter Vergleich der beiden Färbungen von eigentlichem Testansatz und Kontrollansatz somit auch auf digitaler Weise möglich, d.h. der Differenzwert zwischen den Ergebnissen des/jedes eigentlichen Testansatzes und denjenigen des/jedes Kontrollansatzes kann mit digitalen Auswerteprogrammen ermittelt werden, wodurch die Analyse von histochemischen Proben insgesamt weiter erleichtert und beschleunigt und gleichzeitig verlässlicher und sicherer ist.
Bevorzugte Ausführungsvarianten des erfindungsgemäßen Verfahrens bestehen deshalb auch darin, dass die Detektierung der Reaktionssignale in Kontrollansatz (RS-K) und eigentlichem Testansatz (RS-T) und/oder die Subtrahierung (RS-T minus RS-K) und Differenzanzeige teilweise automatisch oder vollautomatisch erfolgt.
Falls der Nachweistest ein immunhistochemischer Test ist, werden die Testergebnisse, d.h. die Ergebnisse der immunhistochemischen Färbungen, digital erfasst und ausgewertet werden, d.h. die Detektierung der Reaktionssignale in Kontrollansatz (RS-K) und eigentlichem Testansatz (RS-T) erfolgt digital, die Subtrahierung (RS-T minus RS-K) erfolgt teilweise automatisch oder vollautomatisch, und die Differenzanzeige digital erfolgt.
Falls der Nachweistest ein Immunfluoreszenztest ist, werden die Testergebnisse, d.h. die Ergebnisse der Immunfluoreszenzen digital erfasst und ausgewertet werden, d.h. die Detektierung der Emissionssignale in Kontrollansatz (RS-K) und eigentlichem Testansatz (RS-T) erfolgt digital, die Subtrahierung (RS-T minus RS-K) erfolgt teilweise automatisch oder vollautomatisch, und die Differenzanzeige erfolgt digital.

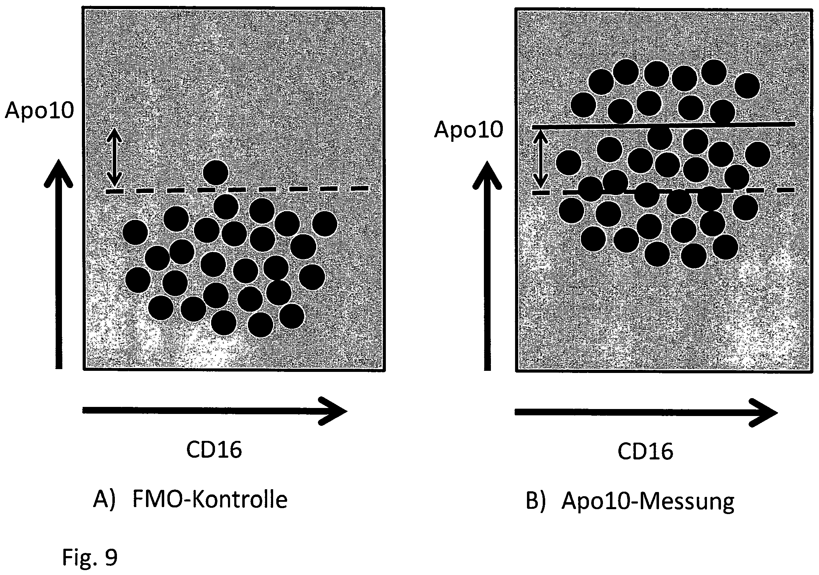

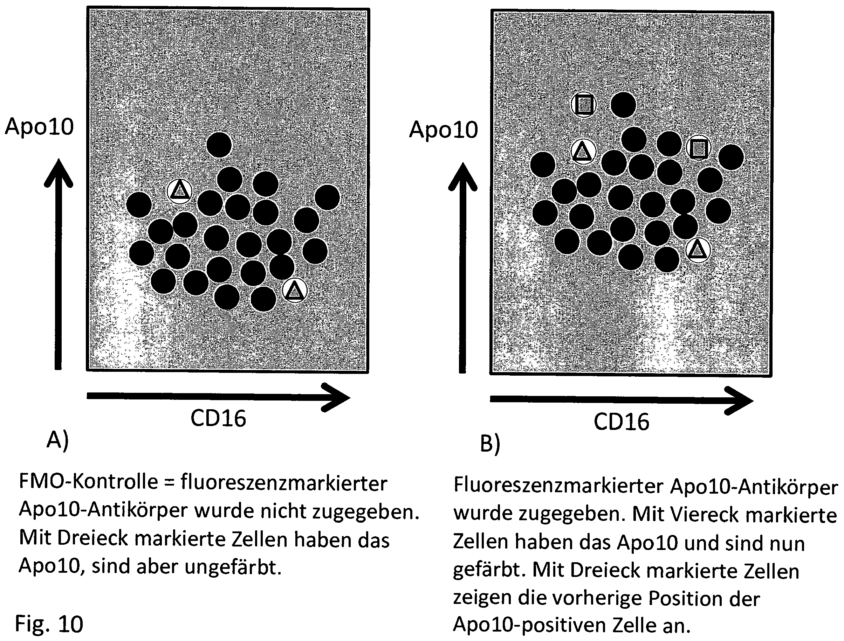

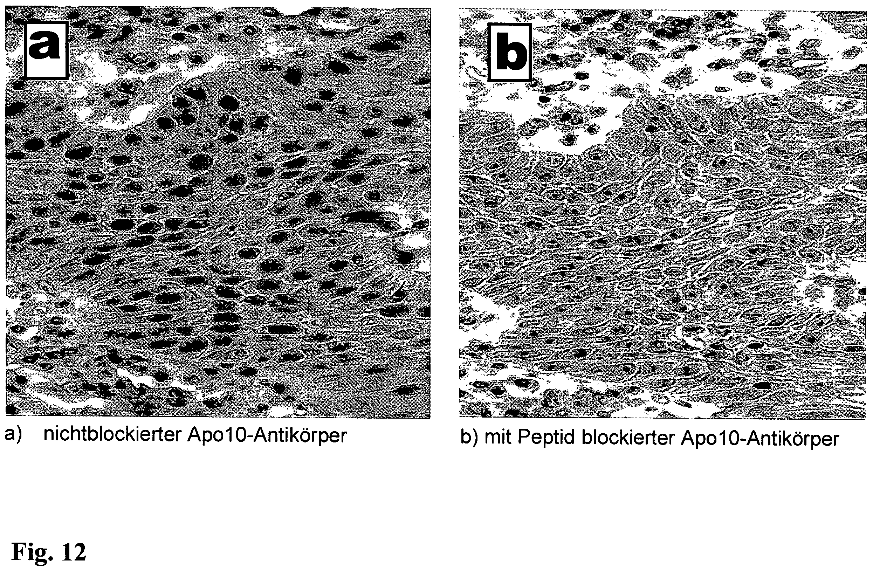

Die Erfindung und Offenbarung wird im Folgenden anhand von Ausführungsbeispielen und dazugehörigen Figuren näher erläutert.
Die Figuren zeigen:

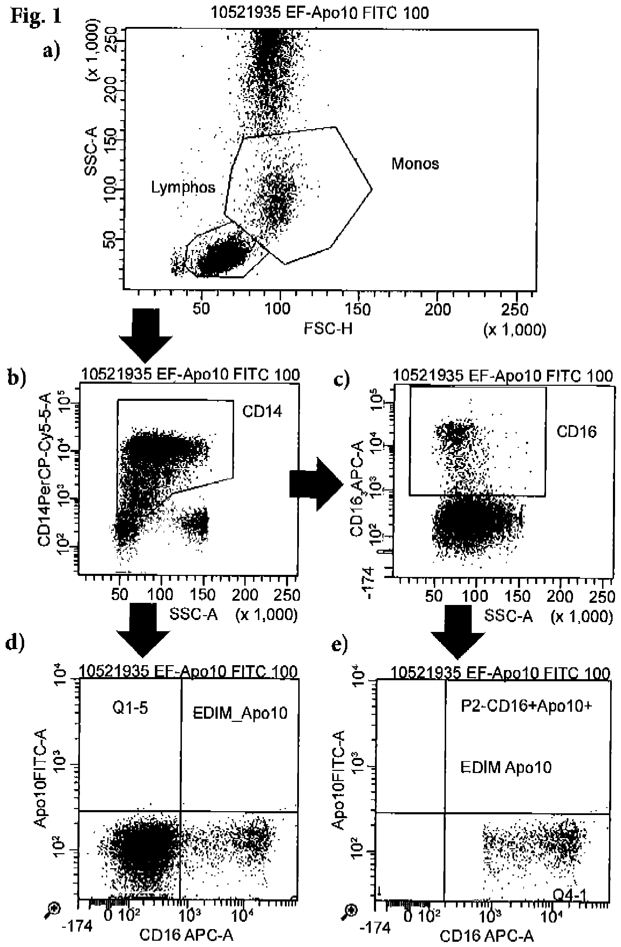

**Fig. 1****: Gating Strategie" zur Identifizierung der Makrophagen**
   **a)** Zur Identifizierung der Monozyten (hier 'Monos' genannt) werden alle Leukozyten sowohl gegen ihre Granularität (SSC-A), als auch gegen die Größe (FSC-H) aufgetragen.
   **b)** Nur die so charakterisierten Zellen werden auf die Anwesenheit des Oberflächenmarkers CD14 hin überprüft. Dazu zählen sowohl die hellsten (= hochpositiven), in der oberen, ovalen Punktwolke und die intermediär hellen (= normal positiven) Zellen. Um diese wird zur weiteren Charakterisierung eine Region ('CD 14' genannt) gelegt.
   **c)** Diese wird zusätzlich auf die Anwesenheit des Oberflächenmarkers CD16 hin überprüft. Hierzu zählen die hellsten (= hochpositiven) in der oberen, ovalen Punktwolke, und die intermediär hellen (= normal positiven) Zellen. Um diese wird zur weiteren Charakterisierung ebenfalls eine Region (CD16 genannt) gelegt.
   **d)** Alle sowohl als 'Monos' als auch als 'CD14' identifizierten Zellen werden auf die intrazelluläre Anwesenheit des Apo10-Antikörpers hin untersucht - zwecks Optimierung der subjektiven Cutoff-Einschätzung.
   **e)** Die in den Regionen 'Monos' als auch in ,CD14' enthaltenen Zellen, die zusätzlich den Oberflächenmarker CD16 tragen, gelten als 'echte Makrophagen'. Sie werden durch den waagerecht liegenden Cuttoff getrennt und damit die als 'Apo10-positive Zellen' bezeichneten Zellen identifiziert (CD14-positiv, CD16-positiv, Apo10-positiv). Als Ergebnis der Messung wird der 'Epitop Detektion in Monozyten/Makrophagen' (EDIM)-Wert als die relative Menge von CD14+/CD16+ positiven Makrophagen dargestellt, die Apo10-positiv sind (im Vergleich zu der Gesamtmenge des CD14/CD16 positiven Makrophagen).

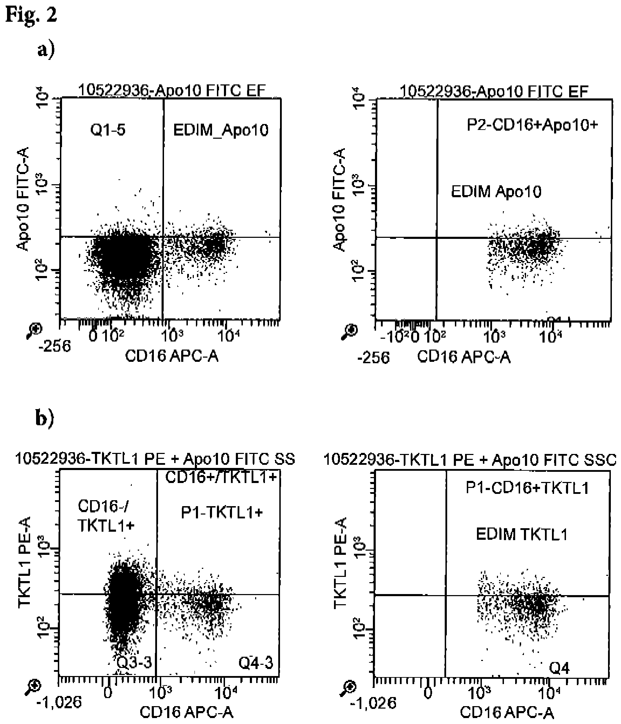

**Fig. 2****: Cutoff-Ermittlung an Blutprobe**
   **a) Antikörper Apo10 (FITC) EDIM-Färbung**
      Der Anteil an Apo10-positiven Makrophagen (Apo10-positiv, CD14-positiv, CD16-positiv) im rechten Teilbild (rote Signalpunkte), d.h. der Makrophagen, die im FITC-Messkanal ein Lichtsignal geben, liegt bei 16,7% oberhalb des waagerechten Cutoffs (Trenngrenze). Im linken Bild sind die hier nicht weiter interpretierten Monozyten zu sehen. Dies gilt auch für die folgenden Abbildungen.
   **b) Antikörper TKTL1 (PE) EDIM-Färbung**
      Der Anteil an TKTL1-positiven Makrophagen (TKTL1-positiv, CD14-positiv, CD16-positiv) im rechten Bild (rote Signalpunkte), d.h. der Makrophagen die im PE-Messkanal ein Lichtsignal geben, liegt bei 17,2% oberhalb des waagerechten Cutoffs (Trenngrenze).

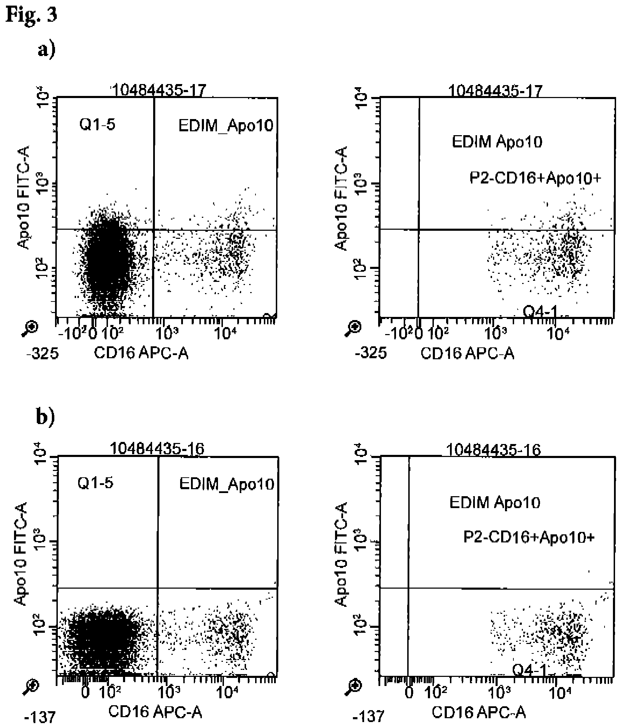

**Fig. 3**: **Relation zwischen Messung und FMO-Kontrolle bei Blutprobe**
   **a) Antikörper Apo10 (FITC) Einzelfärbung**
      Der Anteil an Apo10-positiven Makrophagen (Apo10-positiv, CD14-positiv, CD16-positiv) im rechten Teilbild (rote Signalpunkte), d.h. der Makrophagen die im FITC-Messkanal ein Lichtsignal geben, liegt bei 16,7% oberhalb des waagerechten Cutoffs (Trenngrenze).
   **b) FMO Kontrolle (ohne FITC Antikörper)**
      Der Anteil an positiven Makrophagen im rechten Bild (rot eingezeichnet) liegt deutlich entfernt von der ungefärbten Makrophagenwolke bei nur 0,8% oberhalb des waagerechten Cutoffs. Der Cutoff wurde von der Messung (a) auf die FMO Kontrollmessung übertragen. Dies gilt für die folgenden FMO-Kontroll-Abbildungen ebenfalls.

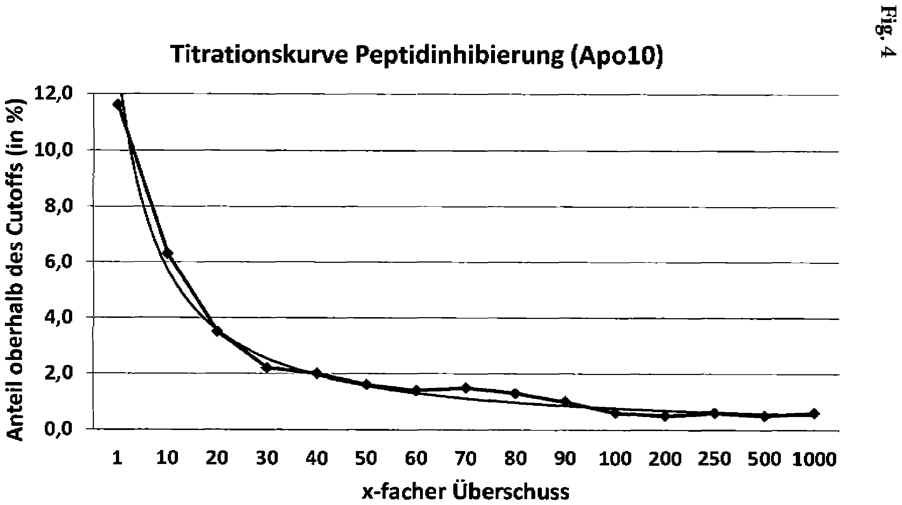

**Fig. 4****: Titrationskurve**
   Färbung von Patientenblut mit 1-facher bis 1000-facher Konzentration des molaren Überschusses von Peptid zu Antikörper Apo10. Auf der x-Achse ist der ansteigende Anteil an Peptid-Überschuss (molarer Überschuss Peptid zu Antikörper) dargestellt, auf der y-Achse der relative Wert der Apo10-positiven Makrophagen oberhalb des Cutoffs (in %).
**Fig. 5****: Parallelmessung mit unblockiertem und blockiertem Apo10-Antikörper**
   **a)** Kontrollansatz mit 10-fachem Überschuss an Blockerpeptid
   **b)** Kontrollansatz mit 500-fachem Überschuss an Blockerpeptid
**Fig. 6****: Schematische Darstellung der durchflusszytometrischen Analyse von zwei Zellen mit unterschiedlich starkem Apo10-(Fluoreszenz-)Signal.**
   Die X-Achse gibt das CD16-Signal wieder; Y-Achse gibt das Apo10-Signal wieder.
   **A)** Zellen mit unblockiertem Apo10-Antikörper
   **B)** Zellen mit blockiertem Apo10-Antikörper
**Fig. 7****: Schematische Darstellung der durchflusszytometrischen Analyse von zwei Zellen mit unterschiedlich starkem Apo10-(Fluoreszenz-) Signal und Darstellung des Ausmaßes der Reduzierung des Apo10-Signals**
   Die X-Achse gibt das CD16-Signal wieder; Y-Achse gibt das Apo10-Signal wieder.
   **A)** Zellen mit unblockiertem Apo10-Antikörper
   **B)** Zellen mit blockiertem Apo10-Antikörper. Die Länge der Pfeile (nach unten) entspricht dem Ausmaß des Absinkens des Apo10-Signals nach Blockierung des Antikörpers.

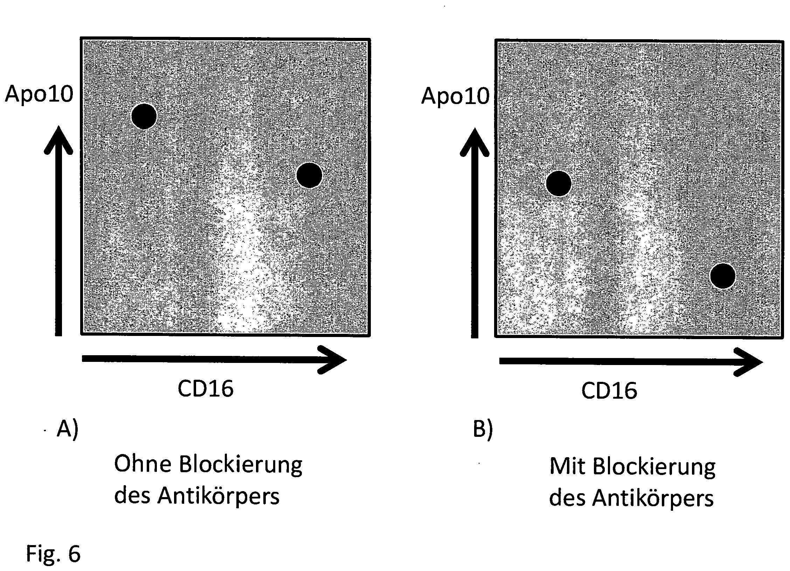

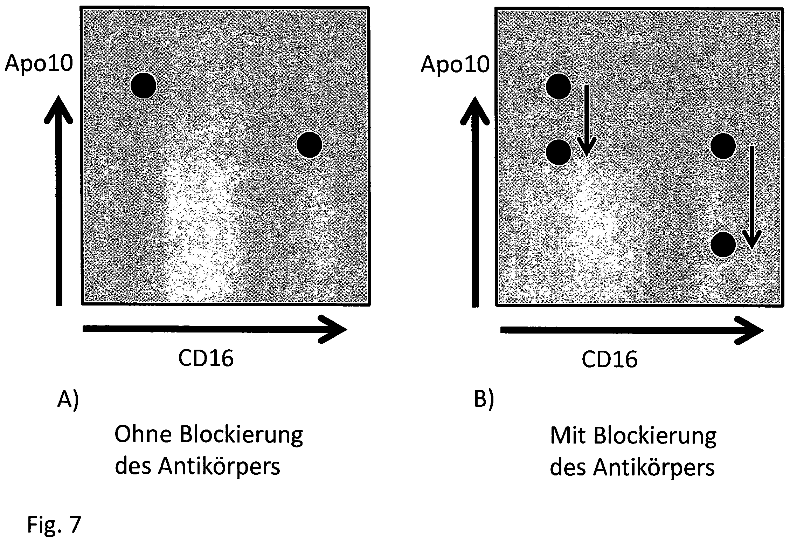

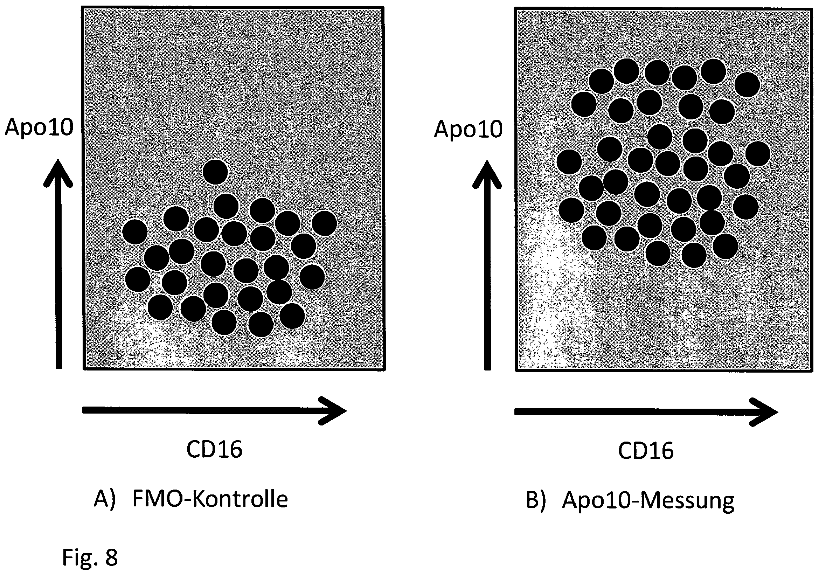

**Fig. 8****: Schematische Darstellung analog** **Fig. 6** **und** **Fig. 7** **für die Analyse von ("eigentlichem") Testansatz und FMO-Kontrolle**
   **A)** Zellen der FMO-Kontrolle (ohne Apo10-Antikörper-Zugabe)
   **B)** Zellen des ("eigentlichen") Testansatzes nach Inkubation und Färbung (Markierung) mit dem fluoreszenzmarkierten Apo10-Antikörper
**Fig. 9****: Darstellung von** **Figur 8** **mit gelegtem Cutoff**
   **A)** Zellen der FMO-Kontrolle (ohne Apo10-Antikörper-Zugabe)
   **B)** Zellen des ("eigentlichen") Testansatzes nach Inkubation und Färbung (Markierung) mit dem fluoreszenzmarkierten Apo10-Antikörper. Die gestrichelte Linie markiert den Cutoff oberhalb der FMO-Zellwolke
**Fig. 10****: Schematische Darstellung der durchflusszytometrischen Analyse von Zellen nach Inkubation mit fluoreszenzmarkiertem Apo10-Antikörper ("eigentlicher" Testansatz) und Zellen einer FMO-Kontrolle.**
   Zellen, die das Apo10-Antigen tragen, sind als Kreis mit innerem Dreieck ( 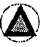 ) dargestellt. Zellen, die das Apo10-Antigen tragen und mit dem fluoreszenzmarkierten Apo10-Antikörper markiert sind und entsprechende Fluoreszenzsignale aussenden, sind hier jeweils als Kreis mit innerem Quadrat ( 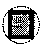 ) dargestellt.
   **A)** Messwerte der der FMO-Kontrolle (ohne Apo10-Antikörper-Zugabe). Apo10-Antigen-positive Zellen (Kreis mit Dreieck) sind vorhanden, werden aber nicht detektiert.
   **B)** Zellen nach Inkubation und Färbung (Markierung) mit dem fluoreszenzmarkierten Apo10-Antikörper. Apo10-Antigen-positive Zellen sind nun durch gebundenen Apo10-Antikörper gefärbt (Kreis mit Quadrat). Die hier dargestellten beiden Kreise mit Dreieck ( 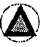 ) geben die vorherige Position der beiden Apo10-Antigen-tragenden Zellen gemäß Fig. 10A an.

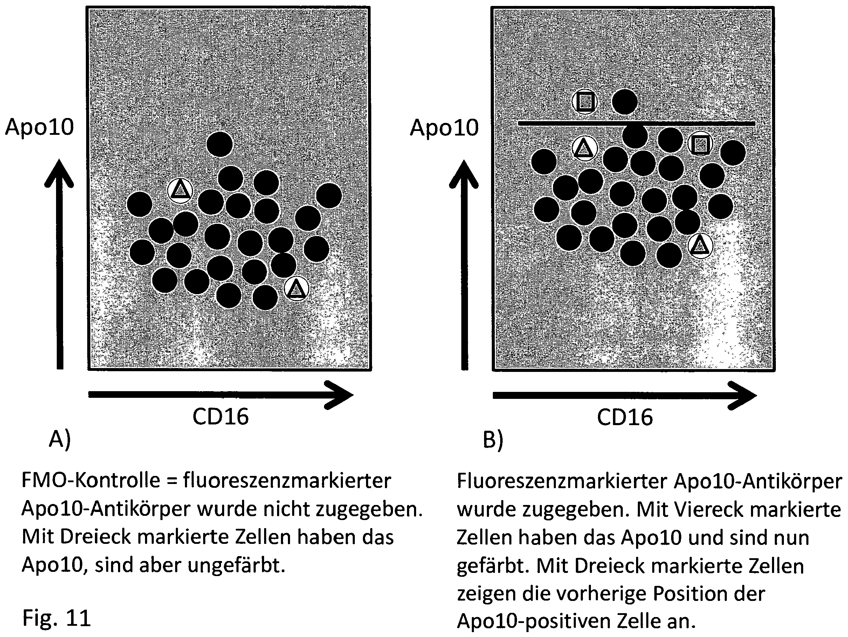

**Fig. 11****: Darstellung gemäß** **Figur 10** **mit gelegtem Cutoff**
   **A)** Zellen der FMO-Kontrolle (ohne Apo10-Antikörper-Zugabe)
   **B)** Zellen des ("eigentlichen") Testansatzes nach Inkubation und Färbung (Markierung) mit dem fluoreszenzmarkierten Apo10-Antikörper
**Fig. 12****: Gewebeschnitte von Mundhöhlenkarzinom nach Inkubation mit dem Detektionsantikörper Apo10 zur Identifizierung von Tumorzellen, die einen erhöhten Anteil DNaseX-Protein mit Apo10-Epitop im Zellkern aufweisen.**
   **a)** Färbung mit nicht-blockiertem Apo10-Antikörper
   **b)** Färbung mit dem Zielepitop/Zielpeptid blockierten Apo10-Antikörper

### Vergleichendes Beispiel 1: Anwendung des offenbarten Verfahrens an einer Blutprobe

Für die Analyse einer Probe, z.B. einer Blutprobe (eines Patienten) sind somit wenigstens zwei Parallelansätze und Parallelmessungen erforderlich: wenigstens ein "eigentlicher Testansatz" und wenigstens ein "Kontrollansatz".

Diese beiden Ansätze unterscheiden sich nur darin, dass in dem "Kontrollansatz" die Bindedomäne(n) des/der Detektionsantikörper(s) blockiert ist/sind, wodurch eine spezifische Bindung unterbunden wird.
Aus dem Vergleich der Messergebnisse beider Ansätze lässt sich das Ausmaß der unspezifischen Bindungen bestimmen, die z.B. vom konstanten Anteil des Detektionsantikörpers oder von einem Zweitantikörper oder von an die Antikörper gekoppelten Farbstoffen oder Enzymen ausgehen.
Bei intrazellulären Färbungen in der Immunzytologie mittels intrazellulär bindender Detektionsantikörper lässt sich mit dem offenbarten Verfahren auch der Anteil der Antikörper bzw. des davon ausgehenden Signals bestimmen, der zwar nicht gebunden, aber auch nicht aus der Zelle ausgewaschen wurde und damit unspezifisch in der Zelle verblieben ist.
Die Variabilität von unspezifischen Bindungen, oder Bindungen, die zwar spezifisch von Strukturen des Antikörpers unabhängig von der eigentlichen Bindedomäne generiert werden, lassen sich ebenfalls mit dem offenbarten Verfahren individuell bestimmen.
So kann zum Beispiel das Ausmaß der Bindung des konstanten Anteils des Detektionsantikörpers mit bestimmten Immunzellen, die sich in Folge einer immunologischen Reaktion im Körper verändert haben, bestimmt werden und auch solche falsch positiven Signale können erkannt und eliminiert werden.

Zwar geht mit dem offenbarten Testverfahren unter Umständen ein höherer Verbrauch an Reagenzien und Testmaterial einher, da sich die Probenmenge (Gewebeproben, Blut) aufgrund des parallelen Kontroll-Ansatzes verdoppelt. Dieser Mehraufwand wird jedoch mehr als kompensiert durch extrem spezifische Signale und eine damit verbundene höhere Sicherheit der Diagnose und eine höhere Sensitivität.

### Vergleichendes Beispiel 2: Anwendung des offenbarten Verfahrens an einer Gewebeschnittprobe

Für die Analyse einer Gewebeschnittprobe (eines Patienten) sind wenigstens zwei Parallelansätze und Parallelmessungen erforderlich: wenigstens ein "eigentlicher Testansatz" und wenigstens ein "Kontrollansatz".

Die Handhabung und Behandlung dieser wenigstens zwei Testansätze erfolgt wie unter Beispiel 1 beschrieben.

### Beispiel 3: Nachweistest mit zwei oder mehr Detektionsantikörpern

Die offenbarte Form der Paralleltestung kann selbstverständlich auch mit zwei oder mehr Detektions-Antikörpern durchgeführt werden, die mit den jeweiligen Zielpeptidsequenzen (oder anderen Zielstrukturen) blockiert sind. Das Signal der Messung ohne Peptidkompetitierung wird dabei von der Messung mit der zeitgleichen Kompetitierung mit mehreren Peptiden abgezogen, um das spezifische Signal zu bestimmen, das durch die Bindung des/der Antikörper mit der Bindedomäne generiert wird.

### Beispiel 4: Anwendung des erfindungsgemäßen Verfahrens in der Durchflusszytometrie

Die Nutzung der Durchflusszytometrie erlaubt eine quantitative Bestimmung fluoreszenzmarkierter Partikel, insbesondere auch fluoreszenzmarkierter Zellen (Fluorescence Activated Cell Sorting, "FACS").
Die fluoreszenzmarkierten Zellen werden mittels eines Flüssigkeitsstroms an einem oder mehreren Lasern vorbei geführt, wobei die Zellen mit der/ den jeweiligen Anregungswellenlänge(n) bestrahlt werden. Daraufhin emittieren die Zellen Licht anderer Wellenlänge. Das emittierte Licht ist proportional zur Menge der gebundenen Antikörper. Die Daten werden dann an ein Auswerteprogramm weitergeleitet und in Diagrammen angezeigt.
Zu den häufigsten und einfachsten durchflusszytometrischen Anwendungen gehört die Analyse von Oberflächenstrukturen auf Einzelzellen, wobei diese Oberflächenstrukturen mit Hilfe von fluoreszenzmarkierten Antikörpern, die an diese Strukturen binden (sogenannte "Detektionsantikörpern"), nachgewiesen werden. Bei den nachweisbaren Epitopen solcher Detektionsantikörper kann es sich neben Proteinepitopen auch um andere Strukturen wie zum Beispiel Zuckerstrukturen, Fette, oder Modifikationen von Proteinen wie Phosphatgruppen handeln.

Eine andere Anwendung der FACS ist der Nachweis von intrazellulären Molekülen wie DNA und RNA, oder von intrazellulären Proteinepitopen. Das spezifische, von der Zielstruktur stammende Signal, wird in der Regel durch den Vergleich mit Kontrollzellen bestimmt. Hierdurch lassen sich dann z.B. Aussagen über den Zellzyklus und die Apoptose treffen, oder bestimmte Zelltypen detektieren wie z.B. normale, gesunde Zellen oder Tumorzellen oder maligne Tumorzellen (Krebszellen).

Neben der Bestimmung von Epitopen auf und in Zellen, ist es mit Hilfe der Durchflusszytometrie auch möglich, Strukturen in Lösungen wie z.B. Zellkulturüberstände oder Serum zu bestimmen. Hierzu werden Beads (oder andere Träger) mit einem oder mehreren fluoreszenzmarkierten Antikörper bestückt und der zu untersuchenden Probe zugegeben, so dass das/die betreffende(n) Antigen(e) (z.B: Interleukine) aus der Probe an den Bead-fixierten Antikörper anbinden.

### Beispiel 5: Anwendung des erfindungsgemäßen Verfahrens in der Durchflusszytometrie mit den Antikörpern 'Apo10' und 'TKTL1' an Blutproben

Mit Hilfe der Durchflusszytometrie sollen intrazelluläre Strukturen in Immunzellen (phagozytierende Immunzellen wie Monozyten/Makrophagen) nachgewiesen werden.

Dazu werden zum einen Antikörper ausgewählt, die spezifisch die Oberfläche der zu untersuchenden Immunzellen markieren (extrazelluläre Strukturen), um diese identifizieren zu können. Zum anderen werden Antikörper ausgewählt, die intrazelluläre Strukturen in den Immunzellen nachweisen. Die Antikörper 'Apo10' und 'TKTL1' sind gegen intrazelluläre Epitope gerichtet.
Die Antikörper sind mit verschiedenen fluoreszierenden Farbstoffen (Fluorochromen) gekoppelt, die innerhalb der Durchflusszytometrie nach Anregung mit Laserlicht unterschiedlich leuchten (emittieren) und sich so identifizieren lassen. Über die Emission wird die Bindung bzw. die Präsenz des Antikörpers an oder in Zellen sichtbar gemacht (monitoriert).

Um Strukturen innerhalb (intrazellulär) in den ausgewählten Zellen nachzuweisen, wird ein Permeabilisierungspuffer verwendet, der Löcher in der Zellmembran generiert, um Antikörper in die Zellen zu schleusen.

Um den Anteil unspezifischer Signale zu bestimmen, der im vorliegenden Fall aufgrund intrazellulärer Färbungen mit Patientenblut mit "klebrigen" Monozyten und Makrophagen besonders hoch sein kann, werden Parallelexperimente ("Kontrollansätze") durchgeführt, bei denen die (Detektions-)Antikörper 'Apo10' und/oder 'TKTL1' vor ihrem Einsatz an ihren Bindedomainen jeweiligs mit einem "Blocker-Peptid" abgesättigt wurden, so dass sie ihr Ziel-Epitop nicht mehr binden und folglich nicht mehr detektieren können.

Die Testergebnisse, die mit den blockierten (Detektions-)Antikörpern generiert werden (diese "Kontrollwert") werden mit demjenigen Testergebnissen verglichen, die mit den unblockierten Antikörpern 'Apo10' und/oder 'TKTL1' generiert werden (den "eigentlichen Testwerten"). Die Differenz zwischen beiden, d.h. (eigentliche) Testwerte minus Kontrollwerte, gibt den Wert an, der für die spezifische Bindung des Antikörpern'Apo10' bzw. des Antikörpers 'TKTL1' an die Zielstruktur steht.

Als ein solches "Blocker-Peptid" wird hier im Beispiel jeweils das Peptid eingesetzt, das bei der Generierung des betreffenden Antikörpers zu Immunisierung der Antikörperproduzierenden Zellen eingesetzt wurde und dessen Aminosäuresequenz folglich identisch mit oder sehr ähnlich der Aminosäuresequenz des Ziel-Epitops ist.

Die Durchführung der Tests ist nachfolgend beschrieben:

### Material und Methoden

Kit: IntraPrep von Beckman Coulter (# A07803);
Antikörper: (außer Apo10) von BD Biosciences, Heidelberg

**Handhabungsschema:**

| Tube | FITC | PE | PerCP | APC |
|---|---|---|---|---|
| 1 (FMO) | | | CD14 (# 345786) | CD16 (# 561304) |
| 2 (EDIM-Färbung) | | | CD14 (# 345786) | CD16 (# 561304) |
| 3 (Peptid-Blockierung | | | CD14 (# 345786) | CD16 (# 561304) |

### Handhabungsprotokoll:

Beschriftung der FACS-Röhrchen (z.B. Round-Bottom Tubes, 1000x5ml von BD, # 352008); Zugabe der jeweiligen extrazellulären Antikörper in Menge laut Herstellerempfehlung; Zugabe von 100 µl EDTA Vollblut, mischen; 15 Minuten Inkubation bei Raumtemperatur und lichtgeschützt; Zugabe von 100 µl IntraPrep Reagenz 1 je Röhrchen und sofort mischen; 15 Minuten Inkubation bei Raumtemperatur und lichtgeschützt;
Parallel jetzt starten mit der Ko-Inkubation des intrazellulären Antikörpers mit Blocker-Peptid (30 Minuten bei Raumtemperatur); erstes Waschen; Zugabe von 4 ml Cell Wash (BD, # 349524) bei Raumtemperatur; Zentrifugation bei 300 g (= 1400 rpm) für 5 Minuten, Überstand gut abkippen; das Pellet vorsichtig mittels Pipette blasenfrei resuspendieren; Zugabe von 100 µl IntraPrep Reagenz 2 je Röhrchen, ! nicht schütteln !; 5 Minuten Inkubation bei Raumtemperatur und lichtgeschützt, danach kurz schütteln;
Zugabe des jeweiligen intrazellulären Antikörpers gemäß folgendem Handhabungsschema und mischen:

| Tube | FITC | PE | PerCP | APC |
|---|---|---|---|---|
| 1 (FMO) | | | | |
| 2 (EDIM-Färbung) | Apo10 (Tavartis) | TKTL1 (Tavartis) | | |
| 3 (Peptid-Blockierung) | Apo10 (Tavartis) | | | |

Anschließend 20' Minuten Inkubation bei Raumtemperatur und lichtgeschützt; zweites Waschen; Zugabe von 4 ml Cell Wash (BD, # 349524); Zentrifugation bei 300 g (= 1400 rpm) für 5 Minuten; Überstand gut abkippen; drittes Waschen; Zugabe von 4 ml Cell Wash (BD, # 349524); Zentrifugation bei 300 g (= 1400 rpm) für 5 Minuten; Überstand gut abkippen; Zugabe von 200 µl Cell Wash (BD, # 349524);
Die Ansätze sind jetzt bereit für Messungen und Auswertungen.

### Durchführung der FACS Messung

Alle Proben wurden mit einem BD FACSCantoII (BD Biosciences, Heidelberg, Deutschland) analysiert. Für jede Probe wurden mindestens 10.000 relevante Messereignisse aufgenommen. FITC, PE, PerCP und APC Fluoreszenzsignale wurden als logarithmisch verstärkte Daten aufgezeichnet. Die Analyse wurde unter Verwendung der BD FACSDiva Software v6.1. (BD Biosciences , Heidelberg, Deutschland) durchgeführt

### Gating Strategie" zur Identifizierung der Makrophagen (Fig. 1):

Im erstens Schritt, zur Identifizierung der Monozyten (hier 'Monos' genannt), werden alle Leukozyten sowohl gegen ihre Granularität (SSC-A), als auch gegen die Größe (FSC-H) aufgetragen (siehe Fig.la).
Nur die so charakterisierten Zellen werden auf die Anwesenheit des Oberflächenmarkers CD14 hin überprüft. Dazu zählen sowohl die hellsten (d.h. hochpositiven) Zellen, - diese entsprechen der oberen, ovalen Punktwolke - , als auch die intermediär hellen (d.h. normal positiven) Zellen. Um diese hellsten und intermediär hellen Zellen wird zur weiteren Charakterisierung eine Region (CD 14 genannt) gelegt (siehe Fig.1b).
Diese Region wird zusätzlich auf die Anwesenheit des Oberflächenmarkers CD 16 hin überprüft. Hierzu zählen sowohl die hellsten (d.h. hochpositiven) Zellen, - diese entsprechen der oberen, ovalen Punktwolke - , als auch die intermediär hellen (d.h. normal positiven) Zellen. Um diese hellsten und intermediär hellen wird zur weiteren Charakterisierung ebenfalls eine Region (CD16 genannt) (siehe Fig.lc) gelegt.
Alle sowohl als 'Monozyt' als auch als 'CD14-positiv' identifizierten Zellen werden auf die intrazelluläre Anwesenheit des Apo10-Antikörpers hin untersucht - zwecks Optimierung der subjektiven Cutoff-Einschätzung.
Die Makrophagen, die in den Regionen "Monozyt" und "CD14" enthalten sind, tragen damit die Oberflächenmarker CD 14 und CD16. Die so identifizierten Makrophagen werden dann durch den waagerecht liegenden Cutoff (Trennlinie) in zwei Populationen getrennt: Apo10-negative unterhalb des Cutoffs und als Apo10-positive Zellen oberhalb des Cuttoffs. Als Ergebnis der Messung wird der (EDIM)-Wert als die relative Menge von CD14+/CD16+ positiven Makrophagen (in Prozent) dargestellt, die Apo10 enthalten (im Vergleich zu der Gesamtmenge des CD14/CD16 positiven Makrophagen).
Das Ergebnis dieser 'Epitop Detektion in Monozyten/Makrophagen'-(EDIM)-Messung besteht in der Angabe bzw. Darstellung der relativen Menge (in Prozent) von CD14+/CD16+ positiven Makrophagen, die zudem Apo10 enthalten (d.h. auch Apo10-positiv sind), im Vergleich zu der Gesamtmenge der CD14/CD16 positiven Makrophagen.

Makrophagen, bei denen der intrazellulär eingeschleuste Antikörper an Epitope bindet, haben eine höhere Signalstärke als die anderen Makrophagen und gelten als Apo10-positive Zellen

### Cutoff-Ermittlung

### (A) Darstellung der Problematik der korrekten Cutoff-Ermittlung am Beispiel von Patientenblut im "EDIM-Normal"-Test

In diesem Ansatz sind gleichzeitig zwei intrazelluläre Detektionsantikörper, nämlich Apo10 und TKTL1, eingesetzt und hinsichtlich Färbung bzw. Signalgebung untersucht worden.
Die Messergebnisse sind in Fig. 2 (a) und Fig.2 (b) dargestellt.

Fig. 2 (a) zeigt das Ergebnis der Apo10-(FITC)-EDIM-Färbung, d.h. des Antikörper Apo 10-(FITC)-EDIM-Nachweisverfahrens:
Im rechten Teilbild sind die Makrophagen repräsentiert, im linken Teilbild die hier nicht weiter interpretierten Monozyten. Der Anteil an Apo10-positiven Makrophagen, d.h. an Makrophagen, die im FITC-Messkanal ein Lichtsignal geben (rote Signalpunkte), liegt bei 16,7% oberhalb des waagerechten Cutoffs (Trenngrenze).

Fig. 2 (b) zeigt das Ergebnis der TKTL1 (PE) EDIM-Färbung, d.h. des Antikörper TKTL1-(PE)-EDIM-Nachweisverfahrens:
Im rechten Teilbild sind die Makrophagen repräsentiert, im linken Teilbild die hier nicht weiter interpretierten Monozyten. Der Anteil an auffälligen Makrophagen, d.h. an Makrophagen, die im PE-Messkanal ein Lichtsignal geben (rote Signalpunkte), liegt bei 17,2% oberhalb des waagerechten Cutoffs (Trenngrenze).

Es zeigt sich, dass die Zuordnung und Interpretation der (spezifisch) positiven/auffälligen Makrophagen, d.h. das Legen des Cutoffs (der Trenngrenze), aufgrund der vielen unspezifischen Signale sehr schwierig ist und nur dem geübten Fachmann mit viel Erfahrung gelingt. Praktisch nur dieser geübte Fachmann erkennt innerhalb der Makrophagenwolke sehr schmale Bereiche, in denen sich die positiven Makrophagen von den negativen absetzen und legt dort den Cutoff an.

Für eine verbesserte Standardisierung wäre also eine zuverlässige Einschätzung des (unspezifischen) Hintergrundsignals sinnvoll, um so einfacher - und genauer - den Cutoff (Trennlinie zwischen positiven und negativen Makrophagen) legen zu können.

Deshalb wurde hier zunächst eine herkömmliche FMO-Kontrolle durchgeführt.
FMO-Kontrolle
Bei/mit dieser FMO-Kontrolle wird die Eigenfluoreszenz der Zellen bestimmt und zudem die Emission der Farbstoffe der extrazellulären Antikörper im Messkanal desjenigen Farbstoffes, der für die intrazelluläre Färbung genutzt wird, und zwar indem eine Messung ohne den Antikörper für die intrazelluläre Färbung durchgeführt wird. Bei vorliegender FMO-Kontrolle wurde also eine Messung mit Antikörpern gegen CD14 und CD16, aber nicht mit Apo10-Antikörper durchgeführt.

In Fig. 3 ist die Relation zwischen den Messergebnissen der "eigentlichen Testansätze" und den Messergebnissen dieser FMO-Kontrolle wiedergegeben:
Fig. 3 (a) zeigt das Ergebnis der Apo10-(FITC)-Einzelfärbung (d.h. des Antikörper Apo10-(FITC)-EDIM-Nachweisverfahrens ohne zweiten Antikörper TKTL1):
Im rechten Teilbild sind die Makrophagen repräsentiert, im linken Teilbild die hier nicht weiter interpretierten Monozyten. Der Anteil an Apo10-positiven Makrophagen, d.h. an Makrophagen, die im FITC-Messkanal ein Lichtsignal geben (rote Signalpunkte), liegt bei 16,7% oberhalb des waagerechten Cutoffs (Trenngrenze).
Fig. 3(b) zeigt das Ergebnis der FMO Kontrolle (ohne FITC Antikörper):
Im rechten Teilbild sind die Makrophagen repräsentiert, im linken Teilbild die hier nicht weiter interpretierten Monozyten. Der Anteil an Makrophagen oberhalb des Cutoffs beträgt nur 0,8%, und diese liegen deutlich entfernt von der ungefärbten Makrophagenwolke. Der Cutoff wurde hierbei von der Messung (a) auf die FMO Kontrollmessung übertragen.

### Fazit:

Die FMO-Kontrolle ist nicht geeignet, um die Ergebnisse für die Messung mit dem Antikörper zu nutzen. Zusätzlich zu den spezifischen Signalen durch Bindung der Antikörper an die nachzuweisenden spezifischen Strukturen zeigen alle Zellen stärkere Signale gegenüber FMO. Dies kann durch Antikörper verursacht werden, die unspezifisch binden oder die in den Zellen nach dem Waschvorgang persistieren.

Deshalb muss der Cutoff optimalerweise so gelegt werden, dass die Zellen in der Wolke unterhalb des Cutoffs bleiben und die tatsächlich positiven Zellen durch den Cutoff abgetrennt werden.
Genau diese optimale Abtrennung (Cutoff-Legung) erfordert jedoch langfristige Erfahrung im Bereich der Durchflusszytometrie und speziell Erfahrung mit jeder einzelnen Fragestellung wie z.B. dem Nachweis von Apo10 oder TKTL1 in Makrophagen. Diese Besonderheit verhindert bisher die Etablierung der Fragestellung in Routinelaboren und/oder birgt das Risiko, dass falsche Ergebnisse ermittelt und dadurch falsche Befunde generiert werden.

### (C) Parallelmessung mit Apo10-Antikörper und Apo10-Antikörper, der durch das spezifische Peptid blockiert ist

Um den unspezifischen Anteil der Signale zu reduzieren, wurde eine zweite Messung mit einem erfindungsgemäßer Kontrollansatz durchgeführt, der analog zum eigentlichen Testansatz mit den gleichen Komponenten in den gleichen Mengen wie bei diesem eigentlichen Testansatz erstellt wurde, und sich von diesem einzig dadurch unterscheidet, dass der Antikörper an seiner spezifischen Bindungsstelle durch vorherige Inkubation mit dem spezifischen Peptid (Epitop-Peptid der Zielstruktur) blockiert ist. Dieser blockierte Antikörper kann keine spezifische Bindung mehr eingehen, so dass die in diesem Kontrollansatz gemessenen Signale aus unspezifischen Bindungen oder einem Verbleib des Antikörpers in den Zellen (trotz Waschvorgang) resultieren.
Um diesen Wert kann dann die eigentliche Messung (d.h. der/die Messwert(e) des eigentlichen Testansatzes) reduziert und damit korrigiert werden, weil dieser Messwert den Wert der (aller) unspezifischen Signale repräsentiert.

Bevor die eigentliche Messung durchgeführt wurde, muss anhand von Titrationskurven die geeignete (molare) Konzentration des blockierenden Peptids ermittelt werden. Im vorliegenden Beispiel wurde dazu aufsteigende molare Mengen Überschuss (von 1-fach (1x) bis 1000-fach (1000x) dieses Blockerpeptids dem Apo10-Antikörper zugesetzt (1x bis 1000x Mol des Peptids im Verhältnis zu einem Mol Antikörper) und seine Auswirkung auf die unspezifischen Bindungen beobachtet, wobei diese Auswirkung in dem Absinken der Signale der Makrophagenwolke zum Ausdruck kommt.

Die erhaltene Titrationskurve ist in Fig. 4 graphisch dargestellt: Auf der x-Achse ist der ansteigende Anteil an Peptid-Überschuss dargestellt, auf der y-Achse der relative Wert der auffälligen Makrophagen oberhalb des Cutoffs (in %). Der zugrunde gelegte Cutoff wurde dabei in der EDIM-Apo10-Einzelfärbung mit dem nicht-inhibierten Apo10-Antikörper durch den Operator aufgrund der optischen Verteilung der Zellen festgelegt.

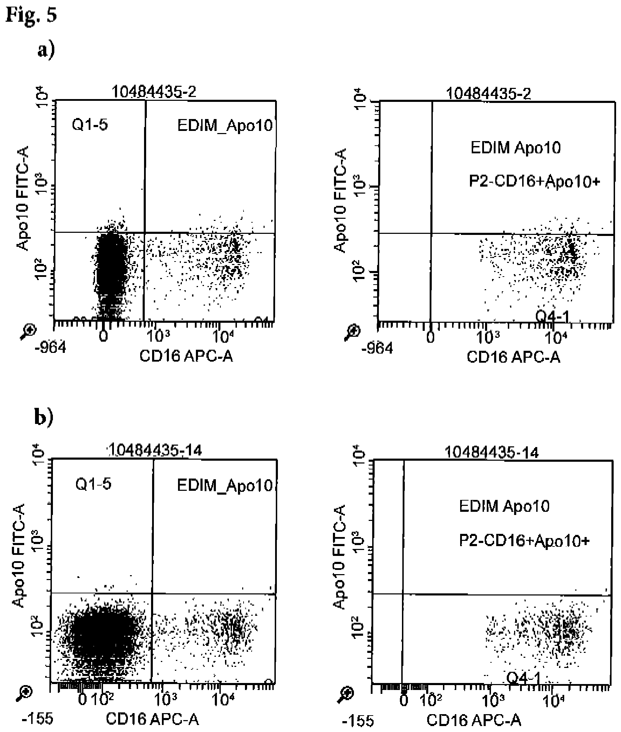

Für die eigentliche Messung wurden zwei Kontrollansätze, einer mit dem 10-fachem Überschuss an Blockerpeptid und einer mit 500-fachem Überschuss an Blockerpeptid eingesetzt. Die Ergebnisse dieser Messungen sind in Fig. 5 dargestellt: Während bei 10-fachem Überschuss des Peptides spezifische Signale oberhalb des Cutoffs erkennbar sind (Fig.5 a), verschwinden diese bei 500-facher Konzentration (Fig.5 b). Dieser 500-fache molare Überschüss des Blockerpeptides im Vergleich zum Apo10-Antikörper ist nötig, um die spezifischen Apo10-Signale zu eliminieren. Die Apo10-Färbung mit 500-fachem Peptidüberschuss (blockierter Apo10-Antikörper) stellt damit eine Kontrolle dar, deren Signale sich aus der Eigenfluoreszenz der Zellen, unspezifisch gebundenen Antikörpern und in den Zellen persistierender Antikörpern nach dem Waschvorgang zusammensetzen und damit die unspezifischen Signale anzeigt. Durch Abzug dieser unspezifischen Messresultate vom Messergebnis, das mit dem unblockierten Apo10-Antikörper generiert wurde, ist die Bestimmung der spezifischen Messsignale möglich.

### Fazit:

Mit dem erfindungsgemäß Verfahren steht ein Auswerteverfahren, obendrein ein automatisierbares Auswerteverfahren zur Verfügung, bei dem jeweils eine Messung mit blockiertem Antikörper von der Messung mit unblockiertem Antikörper abgezogen wird, um so die spezifischen Messsignale zu bestimmen.
Hierzu muss eine Konzentration des Peptides gewählt werden, um spezifische Signale komplett zu unterdrücken. Alle dann noch messbaren Signale entstehen durch Eigenfluoreszenzen der Zellen, durch unspezifische Bindungen des Antikörpers (der konstanten Region des Antikörpers) oder durch in der Zelle verbliebene Antikörper, die kein Epitop gebunden haben. Alle diese unspezifischen Signale können mit dem Antikörper bestimmt werden, dessen Bindestellen für spezifische Bindungen durch das Peptid blockiert sind und somit keine spezifische Bindung mehr möglich ist. Der mit dem Peptid blockierte Antikörper gibt damit das Ausmaß der unspezifischen Bindungen wieder. Die Messsignale bei unblockiertem Antikörper setzen sich damit aus unspezifischen und spezifischen Signalen zusammen und sind dabei insgesamt höher als die Messsignale, die in einer FMO-Kontrolle bestimmt werden. Durch Abzug der unspezifischen Signale (Messung mit blockiertem Antikörper) von den Messergebnissen, die mit dem unblockierten Antikörper generiert wurden, können damit die spezifischen Signale ermittelt werden. Die Auswertung kann damit vollautomatisch erfolgen und Auswerter, die die Auswertung aufgrund optischer Informationen durchführen, sind hierzu nicht mehr erforderlich. Hierzu wird auf die Rohdaten zugegriffen, die in der Software eines Durchflusszytometers hinterlegt sind. Diese können dann zur Berechnung z.B. in Exceltabellen übertragen werden, so dass sie einfach weiterzuverarbeiten sind. Hierdurch können die Rohdaten, die mit dem blockierten Antikörper generiert wurden, von den Rohdaten, die mit dem unblockierten Antikörper generiert wurden, verglichen und substrahiert werden, wodurch der Abzug der unspezifischen Signalen von den Gesamtsignalen möglich ist und die spezifischen Signale bestimmt werden können.
Vor Durchführung der Messung muss für jeden Antikörper eine geeignete Konzentration für die Blockade der Bindestelle ausgetestet werden. Die Konzentration ist spezifisch für die Kombination aus Antikörper mit Bindestelle und Blockadereagenz. Dies bedeutet, dass möglicherweise bei einem anderen Antikörper oder anderen Zelltypen ein anderes Verhältnis aus Peptid und Antikörper eingesetzt werden muss. Dieses Verhältnis muss daher für jedes Testverfahren neu bestimmt werden.

### Beispiel 6: Detektionsprinzip bei Anwendung des erfindungsgemäßen Verfahrens in der Durchflusszytometrie mit dem Antikörpern 'Apo10' an Blutproben

Die Testansätze werden wie in Beispiel 5 beschrieben hergestellt. Der/jeder "eigentliche" Testansatz wird mit dem spezifisch detektionsfähigen (unblockierten) Fluoreszenzfarbstoff-gekoppelten Apo10-Antikörper inkubiert, und der/jeder Kontrollansatz wird mit dem (an seiner Bindedomäne mit dem Epitop-Peptid) blockierten (und deshalb nicht mehr spezifisch detektionsfähigen) Fluoreszenzfarbstoff-gekoppelten Apo10-Antikörper inkubiert. Eine FMO-Kontrolle (d.h. ein Parallelansatz zum "eigentlichen" Testansatz aber im Unterscheid zu diesem komplett ohne Apo10-Antikörper) wurde zusätzlich hergestellt.

Figur 6A ist die schematische Darstellung von zwei Zellen, die ein unterschiedlich starkes aufweisen, wenn (falls, sobald) der unblockierte Apo10-Antikörper, an den ein Fluoreszenzfarbstoff gekoppelt ist, zur Detektion im Durchflusszytometer eingesetzt wird. Auf der X-Achse ist das CD16-Signal, auf der Y-Achse das aufgetragen.
In Figur 6B ist dargestellt, dass nach Blockade der Bindedomäne des Apo10-Antikörpers mit dem Ziel-Peptid in beiden Zellen das Apo10-Signal reduziert wird und infolgedessen beide Zellen in der Darstellung (der durchflusszytometrischen Messwerte) nach unten wandern. Das Ausmaß der Reduzierung des Apo10-Signals ist allerdings bei beiden Zellen unterschiedlich. Die Reduzierung des Apo10-Signals ist bei der rechts befindlichen Zelle stärker - sie ist nun deutlich tiefer in der Darstellung zu sehen als die Zelle links. Die Differenz aus Apo10-Signalstärke bei unblockiertem Antikörper und blockierten Antikörper gibt das Ausmaß der spezifischen Signale an, die aufgrund der spezifischen Bindung des Apo10-Antikörpers generiert werden. Je stärker das nach Blockierung des Antikörpers mit dem Peptid absinkt, desto höher ist der Anteil des spezifischen Signals. Die Pfeillänge gibt damit das Ausmaß des Absinkens des Apo10-Signals nach Blockierung des Antikörpers an (siehe Figur 7). Dies ist das Ausmaß der spezifischen ApolO-Signale.

Figur 7A ist identisch mit Figur 6A. Figur 7B entspricht Figur 6B mit zusätzlicher graphischer Darstellung des Ausmaßes der Reduzierung des Apo10-Signals bei beiden Zellen. Das Ausmaß des Absinkens der Apo10-positiven Zellen gibt das Ausmaß der Reduktion des Signals wieder, das durch die Blockade der Bindedomäne des Apo10-Antikörpers mit Peptid erzeugt wird. Die Reduzierung des Apo10-Signals ist bei der rechts befindlichen Zelle stärker. Die Differenz aus Apo10-Signalstärke bei unblockiertem Antikörper und blockierten Antikörper ist bei der rechten Zelle größer, so dass dort ein höheres Ausmaß an spezifischem Signal vorliegt. Obwohl die Apo10-Signalstärke in der linken Zelle höher ist als in der rechten, weist damit die rechte einen höheren Anteil an spezifischem auf. Das Delta zwischen der Messung mit unblockiertem Apo10-Antikörper und blockiertem Apo10-Antikörper wird durch die Pfeillänge in der Abbildung angegeben und zeigt das Ausmaß der spezifischen Apo10-Färbung. Die Pfeile geben damit das Ausmaß der Senkung des Signals aufgrund der Blockade des Antikörpers mit dem Peptid wieder. Durch die Blockade des Apo10-Antikörpers mit dem Peptid lässt sich das Ausmaß der spezifischen Bindungen bestimmen. Gleichzeitig lassen sich damit auch die Zellen identifizieren, die den höchsten Anteil an spezifischem aufweisen - unabhängig von der Stärke des Gesamtsignals. Hierdurch ist es möglich Zellen zu identifizieren, die zwar ein geringes Apo10-Signal aufweisen, doch in denen dieses Apo10-Signal spezifischer ist als in anderen Zellen mit stärkerem Apo10-Signal.

Figur 8 ist die schematische Darstellung von Zellen, die mit CD 14 und CD16 gefärbt wurden und auf der X-Achse gegen CD16 aufgetragen wurden. Analysiert wurden ("eigentliche") Testansätze und FMO-Kontrolle
In Figur 8A sind die Zellen der FMO-Kontrolle (ohne Apo10-Antikörper-Zugabe) dargestellt.
In Figur 8B ist dargestellt, dass durch Zugabe des fluoreszenzmarkierten Apo10-Antikörpers Apo10-Signale detektiert werden. Das steigt aufgrund spezifischer und unspezifischer Signale in den meisten bis fast allen Zellen an, wodurch diese Zellen in der Darstellung nach oben wandern - die Zellwolke wandert nach oben. In diesem Fall setzen sich die Zellen mit sehr viel Apo10-Signal etwas von der Wolke nach oben hin ab. Es tritt damit aber keine separate, Einzelwolke von Apo10-positiven Zellen oberhalb der ursprünglichen FMO-Zellwolke auf

Figur 9 zeigt, wie in einer Darstellung gemäß Figur 8 (Darstellung von Zellen, die mit CD 14 und CD16 gefärbt wurden und auf der X-Achse gegen das CD16 aufgetragen sind) der Cutoff gelegt wird.
In Figur 9A sind die Zellen der FMO-Kontrolle (ohne Apo10-Antikörper-Zugabe) dargestellt.
Die gestrichelte Linie markiert den Cutoff oberhalb der FMO-Zellwolke.
In Figur 9B markiert die gestrichelte Linie die Trennungslinie (Cutoff) oberhalb der FMO-Zellwolke, die in die Zellwolke mit (bzw. nach bzw. bei) der Apo10-Färbung übertragen wurde. Diese von der FMO übertragene Trennungslinie passt aber nicht mehr, da die Zellwolke aufgrund von spezifischen und unspezifischen Signalen durch Zugabe des fluoreszenzmarkierten Apo10-Antikörpers nach oben gewandert ist. Das Apo10-Signal steigt aufgrund spezifischer und unspezifischer Signale in den meisten bis fast allen Zellen, wodurch die Zellen in der Darstellung nach oben wandern - die Zellwolke wandert nach oben. Die Zellen mit sehr viel setzen sich von der Wolke etwas nach oben hin ab. Es tritt damit aber keine separate Einzelwolke von Apo10-positiven Zellen oberhalb der ursprünglichen FMO-Wolke auf, aber aufgrund der Einschnürung bzw. geringeren Dichte der Zellen in einem Bereich wird aufgrund dieses optischen Unterschiedes eine Trennungslinie (durchgezogene Linie) eingefügt, die Apo10-positive von Apo10-negativen Zellen trennen soll. Diese Trennungslinie liegt aber auf einem deutlich höheren Niveau, als dies aufgrund der FMO-Kontrolle abgeleitet werden kann. Als Apo10-positiv wird hierbei eine Zelle angesehen, die sich oberhalb der Trennlinie befindet. Zellen, die durch Zugabe des Apo10-Antikörpers deutlich an Signal gewinnen, allerdings immer noch nicht die durchgezogene Trennlinie überschreiten, werden dabei als negativ bewertet, obwohl dies tatsächlich Apo10-positive Zellen mit spezifischem Apo10-Signal sein können.

Figur 10 ist wieder die schematische Darstellung von Zellen, die mit CD 14 und CD 16 gefärbt wurden und auf der X-Achse gegen das CD16 aufgetragen wurden. Zellen, die das Apo10-Antigen tragen, sind hier jeweils als Kreis mit innerem Dreieck ( 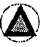 ) dargestellt. Zellen, die das Apo10-Antigen tragen und die durch Bindung des fluoreszenzmarkierten Apo 10-Antikörpes an dieses Antigen markiert sind und die (dieser Bindung) entsprechende Fluoreszenzsignale aussenden, sind hier jeweils als Kreis mit innerem Quadrat ( 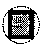 ) dargestellt
In Figur 10A sind die Messwerte der der FMO-Kontrolle (ohne Apo10-Antikörper-Zugabe) dargestellt. Apo10-Antigen-positive Zellen (als Kreis mit Dreieck dargestellt) sind vorhanden, werden hier aber nicht detektiert (weil kein gebundener Apo10-Antikörper vorhanden ist).
In Figur 10B ist dargestellt, dass durch Zugabe des fluoreszenzmarkierten Apo10-Antikörpers die beiden Apo10-Antigen-tragenden Zellen gefärbt werden (als Kreis mit Quadrat). Die in dieser Figur 10B dargestellten beiden Kreise mit Dreieck ( 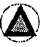 ) geben die vorherige Position dieser beiden Apo10-Antigen-tragenden Zellen gemäß Figur 10 A an. Durch die Zugabe des Apo10-Antikörpers steigt auch die Menge an unspezifischen Signalen, und zwar in allen oder fast allen Zellen. Dies kann z.B. durch die Klebrigkeit des Antikörpers bestimmt bedingt sein. Das in der linken mit dem Viereck markierten Zelle ( 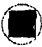 ) steigt durch Zugabe des Apo10-Antikörpers weniger stark an als das in der rechten, mit Viereck markierten Zelle. Obwohl damit die Gesamtstärke des Apo10-Signals in der rechten Zelle niedriger als in der linken ist, ist das Ausmaß des spezifischen Apo10-Signals in der rechten höher als in der links dargestellten Zelle.

Figur 11 zeigt, wie in einer Darstellung gemäß Figur 10 der Cutoff gelegt wird.
Durch Zugabe des fluoreszenzmarkierten Apo10-Antikörpers steigt dabei ein unterschiedlicher Teil der Gesamtzahl der Zellen unterschiedlich stark - die Wolke wandert nach oben. Durch einen Fachmann auf dem Gebiet der Durchflusszytometrie kann aufgrund der Einschnürung bzw. geringeren Dichte der Zellen in einem Bereich eine Zellpopulation im oberen Bereich der Gesamtwolke von Zellen erkennen, die also hohe Apo10-Signale aufweisen und sich etwas von der Restwolke absetzen, aber in der Regel keine separate, von der Hauptwolke getrennte Wolke darstellen. Aufgrund dessen ist es hier nur aufgrund der Erfahrung eines Experten möglich, diese optischen Unterschiede (Einschnürung der Wolke, geringere Dichte im Übergang von Apo10-negativen zu Apo10-positiven Zellen) zu erkennen und hier eine korrekte Trennungslinie einzufügen. Diese Trennungslinie liegt auf einem deutlich höheren Niveau, als dies aufgrund der FMO-Kontrolle abgeleitet werden kann. Dies liegt an dem Anstieg der Gesamtwolke aufgrund einer Kombination aus unspezifischen und spezifischen Apo10-Signalen. Als positiv wird hierbei eine Zelle angesehen, die sich oberhalb der Trennlinie befindet. Zellen, die durch Zugabe des Apo10-Antikörpers deutlich an Signal gewinnen, allerdings immer noch nicht die Trennlinie überschreiten, werden dabei als negativ bewertet. Dies bedeutet, dass nicht allein aufgrund der Zunahme des Apo10-Signals eine Zelle als Apo10-positiv bewertet wird, sondern aufgrund der Zunahme, aber vor allen Dingen aufgrund der Signalstärke. Es werden damit nur die Zellen mit dem stärksten Signal und damit die Zellen oberhalb der Trennlinie (Cutoff) als positiv bewertet. Die Auswertung ist daher nur durch einen Fachmann möglich. Positive Zellen, die nicht über den Cutoff wandern werden als negativ bewertet, obwohl sie positiv sein können und können so das Ergebnis verfälschen.

### Vergleichendes Beispiel 7: Anwendung des erfindungsgemäßen Verfahrens in einem immunohistochemischen Nachweisverfahren mit dem Antikörper 'Apo10' an Gewebeschnitten von Mundhöhlenkarzinom

Der Antikörper 'Apo10' ist ein monoklonaler IgG2a-Antikörper, der gegen das Apo10-Epitop des DNaseX-Proteins gerichtet ist, das bei bestimmten Tumorzellen vermehrt im Zellkern vorhanden ist, und der deshalb als Indikator zur Identifizierung dieser Tumorzellen dienen kann.
Der Antikörper 'Apo10' wurde durch eine Immunisierung von Ratten mit dem DNaseX-Peptid der Aminosäuresequenz CASLTKKRLDKLELRTEPGF generiert. Diese Aminosäuresequenz repräsentiert Aminosäureposition 187 bis 206 des DNaseX-Proteins (Deoxyribonuklease-I-like-1), das aus insgesamt 302 Aminosäuren besteht. Die Gesamtsequenz des DNaseX-Proteins ist unter Accession-Nummer gbAAV38793.1 verfiigbar.
Das Ziel-Epitop des Antikörpers Apo10 ist dieses (dasjenige) Peptid, das bei seiner Generierung zur Immunisierung eingesetzt der Antikörper produzierenden Zellen (in der Ratte) eingesetzt wurde, nämlich ein Peptid mit der Aminosäuresequenz CASLTKKRLDKLELRTEPGF.
Die "'Apo10'-Antikörper-Analyse Färbung" wird an Formalin-fixierten und in Paraffin eingebetteten (FFPE) Humangewebeproben als sogenannte immunhistochemische (IHC) "Apo10-Einzelfärbung" in Kombination mit der Labelled Streptavidin-Biotin-Methode (LSAB-System) durchgeführt.
Hierbei werden die FFPE-Gewebeschnitte zuerst ent-/deparaffiniert und rehydriert, Hitzeinduziert demaskiert, gewaschen und optional mit Peroxidase und/oder Biotin und/oder Serum geblockt. Es folgt die Inkubation mit dem Detektionsantikörper Apo10 als Primärantikörper Antikörperverdünnungsmedium (z.B. Applizierung von 100µl Primärantikörperlösung pro Gewebeschnitt), vorzugsweise über Nacht bei 4° C in einer feuchten Kammer. Die Blockierung der Bindedomäne wird durch Inkubation mit dem 10-fachen molaren Überschuss des Peptides im Vergleich zum eingesetzten Antikörper bei 4°C über Nacht durchgeführt, um so die Primärantikörperlösung des geblockten Antikörpers zu erhalten.

Im Anschluss daran werden die Gebeschnitte gewaschen (z.B. 2x2 Minuten mit Waschpuffer) und mit biotinyliertem Sekundärantikörper (- z.B. Biotin-konj. F(ab')2 Fragments Donkey Anti-Rat IgG (H+L), Verdünnung 1:100 -) 45 Min. bei Raumtemperatur in einer feuchten Kammer inkubiert. Danach werden die Gewebeschnitte wieder gewaschen (z.B. 2x2 Minuten mit Waschpuffer) und mit HRP-konjugiertem Streptavidin (Z.B. LSAB™2 Kit "LABEL") 30 Min. bei Raumtemperatur in einer feuchten Kammer inkubiert.
Es folgt wieder eine Waschung (z.B. 2x2 Minuten mit Waschpuffer) und anschließend eine Inkubation mit Chromogen für HRP (z.B. Biogenex, DAB) für 5-10 Minuten bei Raumtemperatur in einer feuchten Kammer (Inkubationszeit je nach Herstellerangaben). Anschließend wird zuerst mit DAB gefärbt und danach eine Kernfärbung mit Hämatoxilin durchgeführt (z.B. 3 Minuten bei Raumtemperatur, je nach gewünschter Intensität).
Die Färbung wird durch Waschen bzw. Spülen z.B. mit Leitungswasser beendet und die Gewebeschnitte zum Schluss eingedeckt (z.B. in wässrigem Medium oder nach Dehydratation über eine aufsteigende Alkoholreihe/Xylol in dauerhaftem Polymermedium).
Die eingedeckten Gewebeschnitte werden fluoreszenzmikroskopisch analysiert und ausgewertet.
Die Ergebnisse dieser Analyse sind in Fig. 12 dargestellt. Sie zeigen, dass der Apo10-Antikörper das Apo10-Epitop des DNaseX-Proteins im Zellkern von Tumorzellen detektiert (vgl. Fig. 12 a). In Stroma und gesunden Epithelzellen ist dagegen keine Apo10-Färbung und somit kein Detektion des Apo10-Protein-Epitops feststellbar (nicht gezeigt). Die Apo10-Färbung im Zellkern der Tumorzellen ist dann nicht mehr sichtbar, wenn vor der Färbung zu dem Apo10-Antikörper in 10-fachem molaren Überschuss das Zielpeptid/Epitop-Peptid (mit der Aminosäuresequenz CASLTKKRLDKLELRTEPGF) zugegeben wird (vgl. Fig. 12 b).

### Vergleichende immunhistochemische Messung:

Für den Vergleich von zwei immunhistochemischen Messungen, bei denen die immunhistochemische Färbung mit unblockiertem Antikörper mit der Färbung mit blockiertem Antikörper verglichen wird, können Konsekutivschnitte, also zwei aufeinander folgende Schnitte des (Tumor)Gewebes verwendet werden. Hierdurch können durch Substraktion der Signale, die auf zwei aufeinander folgenden Gewebeschnitten generiert wurden, die spezifischen Signale errechnet werden. Diese Methode der Konsekutivschnitte wurde bei Fig. 12 angewendet.
Eine andere Möglichkeit zur vergleichenden Messung von immunhistochemischen Färbungen besteht darin, zunächst den blockierten Antikörper zu dem Gewebeschnitt zuzugeben, um die unspezifischen Signale zu detektieren. Danach kann derselbe Gewebeschnitt mit dem unblockierten Antikörper gefärbt werden, um das Signal zu detektieren, das durch die spezifische Bindung zwischen unblockiertem Antikörper und Zielepitop generiert wird. Hierdurch können durch Substraktion der Signale, die auf demselben Gewebeschnitt generiert wurden, die spezifischen Signale errechnet werden. Die neu hinzukommenden Signale (Signale der zweiten Färbung) stellen hierbei die spezifischen Signale dar, die durch den nichtblockierten Antikörper generiert werden. Eine Modifikation dieser Form der konsekutiven Färbung besteht darin, den blockierten Antikörper in unterschiedlichem Verhältnis im Vergleich zur folgenden Messung mit dem unblockierten Antikörper zuzugeben, so dass zum Beispiel der blockierte Antikörper in 2-fach molarem Überschuss im Vergleich zum unblockierten Antikörper eingesetzt wird. Der Überschuss des blockierten Antikörpers kann noch weiter gesteigert werden, z.B. 3:1 Verhältnis blockierter Antikörper in der ersten Färbung und unblockierten Antikörper in der zweiten Färbung. Dieses Verhältnis kann weiter gesteigert werden auf Verhältnisse von 200:1 oder 500:1 oder noch höher. Mit dieser Titrationsreihe lässt sich ein optimales Verhältnis zwischen blockiertem Antikörper in der einen Messung und unblockiertem Antikörper in der anderen Messung finden, so dass die Stärke der spezifischen Signale so gewählt werden kann, dass diese ausreichend hoch sind. Hierdurch kann die Spezifität noch gesteigert werden, da die unspezifischen Bindungen immer weiter abgesättigt werden und bei einem Vergleich mit dem unblockierten Antikörper noch selektiver die spezifischen Bindungen bestimmt werden können.
Eine weitere Möglichkeit zur vergleichenden Messung von immunhistochemischen Färbungen besteht darin, zunächst den geblockten Antikörper zu dem Gewebeschnitt zuzugeben, um die unspezifischen Signale zu detektieren. Danach kann der blockierte Antikörper durch sanftes Waschen vom Gewebeschnitt entfernt werden. Derselbe Gewebeschnitt wird dann mit dem unblockierten Antikörper erneut gefärbt. Hierdurch werden sowohl spezifische als auch die unspezifischen Signale detektiert. Durch die Substraktion (Differenzbildung) der Signale beider Messungen, die auf demselben Gewebeschnitt generiert wurden, können die spezifischen Signale errechnet werden.

## Patentansprüche

1. Verfahren zur Identifizierung, Quantifizierung und Diskriminierung von spezifischen Signalen gegenüber unspezifischen Signalen in einem Nachweisverfahren, das unter Einsatz wenigstens eines Detektors zum Auffinden und Identifizieren bestimmter Zielstrukturen an Probenmaterial durchgeführt wird, wobei der Detektor Signale zu seinem Nachweis abgibt oder generiert und diese Signale eine Quantifizierung des Detektors ermöglichen, und wobei die spezifischen Signale diejenigen Signale sind, die der Detektor infolge Auffindens und Identifizierens der Zielstruktur abgibt oder generiert,
**dadurch gekennzeichnet,**
- **dass** der Test wenigstens einen eigentlichen Testansatz und wenigstens einen Kontrollansatz umfasst, die parallel durchgeführt werden,
- **dass** der Kontrollansatz dieselben Komponenten in den gleichen Mengen wie der parallel gehandhabte eigentliche Testansatz enthält und sich von diesem eigentlichen Testansatz dadurch unterscheidet, dass die Bindedomäne(n) des Detektors /der Detektoren blockiert ist/sind,
- und **dass** die Gesamtheit der gemessenen Signale im Kontrollansatz von der Gesamtheit der gemessenen Signale im eigentlichen Testansatz subtrahiert wird, wobei die Differenz das spezifische Signal des Detektors /der Detektoren für die nachzuweisende(n) Zielstruktur(en) darstellt.
wobei der Nachweistest ein durchflusszytometrisches Untersuchungsverfahren ist und mittels Durchflusszytometrie durchgeführt wird.

2. Verfahren nach Anspruch 1, **dadurch gekennzeichnet, dass** in dem Kontrollansatz die Bindedomäne(n) des/jedes Detektors mit einer Struktur blockiert ist, die der Zielstruktur gleicht.

3. Verfahren nach Anspruch 1, **dadurch gekennzeichnet, dass** der/die Detektor(en) (ein) Antikörper (Detektionsantikörper), vorzugsweise (ein) monoklonale(r) Antikörper ist/sind.

4. Verfahren nach Anspruch 3, **dadurch gekennzeichnet, dass** in dem Kontrollansatz die Bindedomäne(n) des/der Antikörper(s) (Detektionsantikörper/s) mit einem Peptid blockiert ist/sind, das die Aminosäuresequenz der Zielstruktur aufweist.

5. Verfahren nach Anspruch 3, **dadurch gekennzeichnet, dass** in dem Kontrollansatz die Bindedomäne(n) des/der Antikörper(s) (Detektionsantikörper/s) mit einem Proteinfragment oder einem Protein blockiert ist/sind, das die Ziel strukturumfasst.

6. Verfahren nach Anspruch 3, **dadurch gekennzeichnet, dass** in dem Kontrollansatz die Bindedomäne(n) des/der Antikörper(s) (Detektionsantikörper/s) durch Bindung mit einem oder mehreren Peptid-Kompetitoren blockiert ist/sind, wobei jeder Peptid-Kompetitor mit dem Peptid, das die Aminosäuresequenz der Zielstruktur aufweist, funktions- und wirkungsgleich ist.

7. Verfahren nach Anspruch 3, **dadurch gekennzeichnet, dass** in dem Kontrollansatz die Bindedomäne(n) des/der Antikörper(s) (Detektionsantikörper/s) durch Bindung mit einem oder mehreren Peptid-Kompetitoren und/oder Proteinfragment(en) und/oder Protein(en) blockiert ist/sind, wobei jeder Peptid-Kompetitor mit dem Peptid, das die Aminosäuresequenz der Zielstruktur aufweist, funktions- und wirkungsgleich ist, und jeder Proteinfragment- oder Proteinkompetitor die Zielstruktur umfasst.

8. Verfahren nach einem der Ansprüche 1 bis 7, **dadurch gekennzeichnet, dass** die Detektierung der Reaktionssignale in Kontrollansatz (RS-K) und eigentlichem Testansatz (RS-T) und/oder die Subtrahierung (RS-T minus RS-K) und Differenzanzeige teilweise automatisch oder vollautomatisch erfolgt.

9. Verfahren nach einem der Ansprüche 1 bis 8, **dadurch gekennzeichnet, dass** der Nachweistest ein immunhistochemischer Test ist, und dass die Testergebnisse digital erfasst und ausgewertet werden, nämlich die Detektierung der Reaktionssignale in Kontrollansatz (RS-K) und eigentlichem Testansatz (RS-T) digital erfolgt, und die Subtrahierung (RS-T minus RS-K) teilweise automatisch oder vollautomatisch erfolgt und die Differenzanzeige digital erfolgt.

10. Verfahren nach Anspruch 9, **dadurch gekennzeichnet, dass** der Nachweistest ein Immunfluoreszenztest ist, und dass die Ergebnisse der Immunfluoreszenzen digital erfasst und ausgewertet werden, nämlich die Detektierung der Emissionssignale in Kontrollansatz (RS-K) und eigentlichem Testansatz (RS-T) digital erfolgt, und die Subtrahierung (RS-T minus RS-K) teilweise automatisch oder vollautomatisch erfolgt und die Differenzanzeige digital erfolgt.

## Claims

1. Method for identification, quantification and discrimination of specific signals over unspecific signals in a detection method that is conducted using at least one detector for locating and identifying specific target structures on sample material, wherein the detector submits or generates signals for its determination and these signals allow a quantification of the detector and wherein the specific signals are the signals that were submitted or generated by the detector due to the finding and identification of the target structure, **characterized in,**
- **that** the test comprises at least one test preparation and at least one control preparation, which are handled in parallel
- **that** the control preparation contains the same components in the same amounts as the test preparation handled in parallel and which differs from the actual test preparation in that the binding domain(s) of the detector(s) is/are blocked
- and **that** the total of the signals measured in the control preparation are subtracted from the total of signals measured in the test preparation, wherein the difference constitutes the specific signal of the detector(s) of the cell structure(s) to be determined,
wherein the detection method is a flow cytometric examination method and is conducted via flow cytometry.

2. Method according to claim 1, **characterized in that** in the control preparation the binding domain(s) of the/each detector are blocked with a structure that is similar to the target structure.

3. Method according to claim 1, **characterized in that** the detector(s) is/are (an) antibody (detection antibody), preferably (a) monoclonal antibody.

4. Method according to claim 3, **characterized in that** in the control preparation the binding domain(s) of the antibody/antibodies (detection antibody/antibodies) are blocked with a peptide that exhibits the amino acid sequence of the target structure.

5. Method according to claim 3, **characterized in that** in the control preparation the binding domain(s) of the antibody/antibodies (detection antibody/antibodies) are blocked with a protein fragment or with a protein that comprises the target structure.

6. Method according to claim 3, **characterized in that** in the control preparation the binding domain(s) of the antibody/antibodies (detection antibody/antibodies) is/are blocked through the binding of one or more peptide competitors, wherein each peptide competitor is functionally or effectively equal to the peptide that exhibits the amino acid sequence of the target structure.

7. Method according to claim 3, **characterized in that** in the control preparation the binding domain(s) of the antibody/antibodies (detection antibody/antibodies) is/are blocked through the binding of one or more peptide competitors and/or peptide fragment(s) and/or protein(s), wherein each peptide competitor is functionally or effectively equal to the peptide that exhibits the amino acid sequence of the target structure, and each protein fragment or protein competitor comprises the target structure.

8. Method according to any of the claims 1 to 7, **characterized in that** the detection of the reaction signals in the control preparation (RS-K) and in the actual test preparation (RS-T) and/or the subtraction (RS-T minus RS-K) and the difference display occurs partly automated or fully automated.

9. Method according to any of claims 1 to 8, **characterized in that** the detection test is an immune histochemical test and that the test results are recorded and assessed digitally, namely that the detection of the reaction signals in the control preparation (RS-K) and in the actual test preparation (RS-T) occurs digitally, and the subtraction (RS-T minus RS-K) occurs partly automated or fully automated and the difference display occurs digitally.

10. Method according to claim 9, **characterized in that** the detection test is an immune-fluorescence test and that the results of the immunofluorescence are recorded and assessed digitally, namely that the detection of the emission signals in the control preparation (RS-K) and in the actual test preparation (RS-T) occurs digitally, and the subtraction (RS-T minus RS-K) occurs partly automated or fully automated and the difference display occurs digitally.

## Revendications

1. Procédé pour l'identification, la quantification et la discrimination de signaux spécifiques par rapport à des signaux non spécifiques dans un procédé de détection, qui est réalisé avec utilisation d'au moins un détecteur pour trouver et identifier des structures cibles déterminées sur un matériau d'échantillon, le détecteur émettant ou générant des signaux pour sa détection et ces signaux permettant une quantification du détecteur et les signaux spécifiques étant les signaux que le détecteur émet ou génère suite à la détection et à l'identification de la structure cible,
**caractérisé**
- **en ce que** le test contient un au moins dosage de test en soi et au moins un dosage de contrôle, qui sont réalisés en parallèle,
- **en ce que** le dosage de contrôle contient les mêmes composants, aux mêmes quantités, que le dosage de test en soi manipulé en parallèle et se distingue de ce dosage de test en soi en ce que le(s) domaine(s) de liaison du détecteur est/sont bloqué(s),
- et **en ce que** la totalité des signaux mesurés dans le dosage de contrôle est soustraite de la totalité des signaux mesurés dans le dosage de test en soi, la différence représentant le signal spécifique du/des détecteur(s) pour la/les structure(s) cible(s) à détecter,
le test de détection étant un procédé d'analyse par cytométrie en flux et étant réalisé au moyen d'une cytométrie en flux.

2. Procédé selon la revendication 1, **caractérisé en ce que** dans le dosage de contrôle, le(s) domaine(s) de liaison du/de chaque détecteur est bloqué par une structure qui est identique à la structure cible.

3. Procédé selon la revendication 1, **caractérisé en ce que** le(s) détecteur(s) est/sont un/des anticorps (anticorps de détection), de préférence un/des anticorps monoclonal/monoclonaux.

4. Procédé selon la revendication 3, **caractérisé en ce que** dans le dosage de contrôle, le(s) domaine (s) de liaison du/des anticorps (anticorps de détection) est/sont bloqué(s) par un peptide qui présente la séquence d'acides aminés de la structure cible.

5. Procédé selon la revendication 3, **caractérisé en ce que** dans le dosage de contrôle, le(s) domaine (s) de liaison du/des anticorps (anticorps de détection) est/sont bloqué(s) par un fragment protéinique ou une protéine qui comprend la structure cible.

6. Procédé selon la revendication 3, **caractérisé en ce que** dans le dosage de contrôle, le(s) domaine(s) de liaison du/des anticorps (anticorps de détection) est/sont bloqué(s) par liaison à un ou plusieurs compétiteurs peptidiques, chaque compétiteur peptidique présentant la même fonction et la même action que le peptide qui présente la séquence d'acides aminés de la structure cible.

7. Procédé selon la revendication 3, **caractérisé en ce que** dans le dosage de contrôle, le(s) domaine(s) de liaison du/des anticorps (anticorps de détection) est/sont bloqué(s) par liaison à un(e) ou plusieurs compétiteurs peptidiques et/ou fragment(s) protéinique(s) et/ou protéine(s), chaque compétiteur peptidique présentant la même fonction et la même action que le peptide qui présente la séquence d'acides aminés de la structure cible et chaque compétiteur de fragment protéinique ou compétiteur protéinique comprenant la structure cible.

8. Procédé selon l'une quelconque des revendications 1 à 7, **caractérisé en ce que** la détection des signaux de réaction dans le dosage de contrôle (RS-K) et dans le dosage de test en soi (RS-T) et/ou la soustraction (RS-T moins RS-K) et l'affichage de la différence a lieu de manière partiellement ou totalement automatique.

9. Procédé selon l'une quelconque des revendications 1 à 8, **caractérisé en ce que** le test de détection est un test immunohistochimique et **en ce que** les résultats de test sont enregistrés et évalués de manière numérique, à savoir, la détection des signaux de réaction dans le dosage de contrôle (RS-K) et dans le dosage de test en soi (RS-T) a lieu de manière numérique et la soustraction (RS-T moins RS-K) a lieu de manière partiellement ou totalement automatique et l'affichage de la différence a lieu de manière numérique.

10. Procédé selon la revendication 9, **caractérisé en ce que** le test de détection est un test d'immunofluorescence et **en ce que** les résultats de l'immunofluorescence sont enregistrés et évalués de manière numérique, à savoir, la détection des signaux d'émission dans le dosage de contrôle (RS-K) et dans le dosage de test en soi (RS-T) a lieu de manière numérique et la soustraction (RS-T moins RS-K) a lieu de manière partiellement ou totalement automatique et l'affichage de la différence a lieu de manière numérique.
